# EUROPEAN PATENT SPECIFICATION

(11) **EP 4 368 099 B1**
(45) Date of publication and mention of the grant of the patent: **02.09.2026**
(21) Application number: 24167025.6
(22) Date of filing: 28.02.2022
(51) Int. Cl.: A61B 5/0205, G16H 40/67, G16H 50/20, G16H 20/00

(54) **SYSTEMS AND METHODS FOR REMOTE CLINICAL EXAMS AND AUTOMATED LABELING OF SIGNAL DATA**
SYSTEME UND VERFAHREN FÜR KLINISCHE FERNUNTERSUCHUNGEN UND AUTOMATISIERTE ETIKETTIERUNG VON SIGNALDATEN
SYSTÈMES ET PROCÉDÉS D'EXAMENS CLINIQUES À DISTANCE ET D'ÉTIQUETAGE AUTOMATISÉ DE DONNÉES DE SIGNAL

(30) Priority: 22.04.2021 US 202163201286 P
(43) Date of publication of application: 15.05.2024
(62) Document number(s) of the earlier application(s) in accordance with Art. 76 EPC: 22792663.1 / 4 327 336
(73) Proprietor: Verily Health Inc., Dallas, Texas 75019 (US)
(72) Inventor: KAPUR, Ritu, San Francisco, 94080 (US); BURQ, Maximilien, San Francisco, 94080 (US); RAINALDI, Erin, San Francisco, 94080 (US); MYERS, Lance, San Francisco, 94080 (US); MARKS, William, San Francisco, 94080 (US)
(74) Representative: Mewburn Ellis LLP

(56) References cited:
- US-A1- 2019 183 403
- US-B2- 10 896 756

## Description

### TECHNICAL FIELD

The present invention relates to systems, methods, and devices relating to identification and annotation of signal data associated with wearable sensor devices.

### BACKGROUND

A wearable user device such as a watch may use one or more sensors to sense data representative of physiological signals of a wearer. In some cases, certain sensors may be used (or configured with a different sampling rate) when the wearer performs a predefined action or set of actions requested by the wearable user device. During this time, the sensor data collected may be of varying relevancy to the predefined action or set of actions.

US 2019/0183403 A1 discloses a device for diagnostic measurement by simultaneous multimodal analysis of a subject's head.

### BRIEF SUMMARY

Aspects of the invention are set out in the appended claims.

### BRIEF DESCRIPTION OF THE DRAWINGS

The accompanying drawings, which are incorporated into and constitute a part of this specification, illustrate one or more certain examples and, together with the description of the example, serve to explain the principles and implementations of the certain examples.
FIG. 1 illustrates an example system including a user device for use in implementing techniques related to activity identification and automatic annotating of sensor data from a wearable sensor device, according to at least one example.
FIG. 2 illustrates a system and a corresponding flowchart illustrating a process for identifying activities in and automatically annotating sensor data of wearable sensor devices, according to at least one example.
FIG. 3 illustrates an example flowchart illustrating the process relating to implementing techniques relating to identifying activities and automatically generating annotations, according to at least one example.
FIG. 4 illustrates a diagram including an example sensor and annotated sensor data, according to at least one example.
FIG. 5 illustrates a diagram including example sensor data from the user device at different points in time for identifying activities and generating annotations descriptive of activity performance, according to at least one example.
FIG. 6 illustrates an example flowchart illustrating the process relating to implementing techniques relating to automatically generating annotations for sensor data from a wearable sensor, according to at least one example.
FIG. 7 illustrates an example flowchart illustrating a process related to implementing techniques relating to training a machine-learning model to generate annotations for sensor data from a wearable sensor, according to at least one example.
FIG. 8 illustrates an example architecture or environment configured to implement techniques relating to identifying activities and annotating sensor data associated with the activities, according to at least one example.

### DETAILED DESCRIPTION

Examples are described herein in the context of identifying and automatically annotating sensor data collected by wearable user devices while conducting virtual motor exams (VMEs) on the wearable user devices or performing other activities in a non-structured manner, such as a free-living setting. Those of ordinary skill in the art will realize that the following description is illustrative only and is not intended to be in any way limiting. For example, the techniques described herein can be used to identify and annotate sensor data collected during different types of structured exams, activities, and/or non-structured times, and in some examples, may be implemented on non-wearable user devices. Reference will now be made in detail to implementations of examples as illustrated in the accompanying drawings. The same reference indicators will be used throughout the drawings and the following description to refer to the same or like items.

In the interest of clarity, not all of the routine features of the examples described herein are shown and described. It will, of course, be appreciated that in the development of any such actual implementation, numerous implementation-specific decisions must be made to achieve the developer's specific goals, such as compliance with application- and business-related constraints, and that these specific goals will vary from one implementation to another and from one developer to another.

Parkinson's disease (PD) and other neurological disorders may cause motor disorders. Conventionally, a trained clinician will conduct a motor examination at a clinic or in a patient's (e.g., referred to herein as a user) home to help determine whether the user's symptoms are related to a certain motor disorder, such as Parkinson's disease, and to also track progression of such disorders. For example, during a physical component such as an exam for Parkinson's, the clinician will look at tremors (e.g., repetitive movement caused by involuntary contractions of muscles), rigidity (e.g., stiffness in arms or legs), bradykinesia or akinesia (e.g., slowness of movement and/or lack of movement during regular tasks), and postural instability (e.g., natural balance issues). In some examples, at least some of the examination may be based on the Unified Parkinson's Disease Rating Scale (UPDRS).

Using an example wearable device according to the examples described herein, a user can conduct these (and other types of) exams at home without physician oversight. In this illustrative example, the wearable device includes logic to direct the user's activities, which, in some examples, may require different types of movement or stillness. As described in detail herein, example wearable devices may include multiple sensors that collect sensor data as the user performs these activities. This sensor data can then be processed to derive physiological signals of the user to identify the same or similar observations as a physician would make during an office visit and thereby provide a more complete view of the status of the user over time as opposed to at a single snapshot in time during a clinical visit.

In traditional disease progression measurements (e.g., for running clinical trials), exams have to be done under stringent controlled environmental conditions in a clinic or lab setting, which fails to account for how symptoms affect real world activities in the real world settings and also fails to provide an accurate picture of the symptoms outside of short snapshots of clinical exams. Additionally, dependency on clinic and lab settings can preclude rural areas from having access to disease progression measurements as regular clinical exams and visits are difficult, inconvenient, or impossible. Furthermore, additional real-time biometric and environmental monitoring sensors and mobile applications typically fail to provide sufficient data generation for use in clinical trials or for tracking disease progression because too many variables may impact the sensor readings. Conventional disease progression measurements are collected by a clinician in a clinical setting and provided by a user sharing subjective feedback using paper forms. These procedures result in hurdles that lead to low adoption rates, lost data, unusable data, and prohibitive costs.

The systems and methods described herein resolve the problems above and provide for improvements over existing technologies by automatically generating annotations that are attached to sensor data collected by a user device. The annotations are generated at the user device rather than at a remote device by post-processing the sensor data. The annotations may be generated at the user device by a module that generates and attaches or appends the annotations to passive sensor data (e.g., gathered when a user is not performing a virtual clinical exam). Some annotations may be generated based on user inputs received at the user device that collects the sensor data. The annotations may be validated or verified using metadata and additional sensor data from secondary sensors to ensure consistency in annotation generation and attachment. In some examples, the annotations include context information, such as context data, describing a location, activity, behavior, or other such contextual data. The annotations may also include information related to a user's subjective rating or belief about their experience during an activity (e.g., pain, comfort, sentiment). In some particular cases, the sensor data and the annotations are collected and generated while the user performs a set of tasks that may be probative or instructive for providing information related to disease progression of a particular disease. The annotation generation may take place on the user device, and therefore capture contextual data at the moment of capture and not rely on post-processing to re-create the conditions surrounding the sensor data to attempt to generate the annotations. In this manner, the annotations may capture additional contextual information that may be lost if the data were post-processed out of context and at a later time.

The systems and methods described herein provide an end-to-end system and method for conducting virtual exams and generating meaningful annotations to accompany data gathered during virtual exams as well as during everyday life of a wearer in a free-living environment. The system includes one or more user/wearable devices including sensors for gathering data such as motion and acceleration data, a remote server, a clinical repository, and a variety of interfaces to enable interaction with the system. A wearable device collects raw sensor data from the sensors while a user performs a predefined task and at other times, receives subjective and objective feedback from a wearer via an interface on the devices (e.g., how did you feel during this task, where you walking during this task, etc.). The raw data and the subjective and objective feedback are shared with the remote server.

In some cases, the wearable device and/or the remote server generates annotations describing contexts, predicted scores, predicted subjective comments, and other such data based on the raw sensor data. In some cases, the wearable device generates annotations based on the contextual data, appends the annotations with the sensor data, and conveys a data package to a remote server, the data package including the sensor data and the generated annotations. In this manner, the annotations may be generated at or near real-time and not require post-processing. Some annotations may include corroborative data from biometric signals from the sensor data, contextual information such as whether a medication was taken before or after an activity, what type of activity the wearer is engaging in, and other such information. Annotated and/or un-annotated raw sensor data is stored in the data repository at which a variety of purpose-built algorithms are used to generate additional annotations. The annotated data, such as data from clinical exams or VMEs is used to train a predictive model and, in some cases, as input to the predictive model for scoring the data. The predictive model is able to generate annotations for un-annotated raw sensor data, such as from a free-living setting, to provide a more complete snapshot of the symptoms, progress, and status of the wearer. In some examples, the wearable device and/or the remote server may also determine when to request a VME, clinical visit, or other such exam to receive updated ground truth annotations for calibrating and/or re-training the predictive model.

The annotations to accompany the raw sensor data as generated by the predictive model may be generated by a clinical annotation module that generates and attaches annotations for passive sensor data (e.g., collected when a user is not performing virtual clinical exam or in a clinical setting) and active sensor data (e.g., when the user is performing the virtual clinical exam or in the clinical setting). Some annotations may be generated based on user input received at the device that collected the sensor data, such as a user indication of a start of an activity, a user subjective input after performing a task, or other data such as data indicating the wearer is walking or performing a task that may be similar or identical to a VME task. The annotations may be validated using metadata and other sensor data to ensure the annotations generated by the predictive model are consistent over a period of time, for example to account for subjective fluctuations in ratings provided by clinical professionals or wearers.

In some examples, the annotations may include contextual information (e.g., clinical context, geolocation context, activity context, behavioral context, context for validating supposed truth labels, etc.). Another set of annotations may describe a wearer's subjective belief about the experience (pain, comfort, sentiment). The predictive model or a validation module may cross-validate/confirm a set of annotations by asking redundant questions in an interesting or different way to elicit different responses from a wearer. Yet another set of annotations may include ratings (e.g., pain on scale 1-5). Clinicians may also provide annotations for the raw data. In some examples, the raw sensor data and annotations may be collected while the user performs a set of tasks that would be most probative (e.g., the top 8 tasks that have the largest indicators) of disease progression for a particular disease. The annotation generation can take place on the device or in the cloud, such as on the remote server. Optionally, other data sources such as electronic medical records can be used to train and/or predict on the predictive model.

In some examples, the systems and methods provide for gathering signal data from the wearable sensor during a free-living setting or period of time and to inferring what the user was doing while the signal data was collected. The systems and methods also provide for mapping various tasks identified from the signal data to a task in a particular test, such as a particular test of a VME. This mapping is using a model that has been trained using clinical data. Further still, the model, or another model, may provide annotations such as ratings, predicted subjective feedback, predicted activity identification, predicted contextual data, and other such annotations based on the raw sensor data, and potentially from additional sensor data, such as from a user device or other sensor device in communication with the wearable sensor system and/or the remote sensor. For example, a mobile phone device may provide activity information, location, or other such data to further aid the predictive model in identifying activities performed by the wearer and/or to validate predicted activity identification output by the predictive model.

In a particular example, a user is provided a wearable device such as a watch as part of a disease progression program. The watch may include a set of sensors configured to track various movements, heart rate, etc. of the user and software to conduct various VMEs. The VMEs may be accessed on demand by the user and/or the watch may suggest a suitable time for conducting an exam. In either case, to begin an exam, the user may select a button (e.g., a physical button or graphical user interface ("GUI") element) and the same or a different button to end. The wearable device may generate timestamps to indicate the beginning and the end of the exam, which may be associated with an exam identifier (e.g., an identifier that uniquely identifies the type of exam) and a session identifier (e.g., an identifier that uniquely identifies a session in which the exam was conducted). Additionally, during the exam, the wearable device may instruct multiple sensors to collect sensor data, which may be obtained in the form of signal data. After the exam has concluded or during the exam, the wearable device may determine a context window that represents some period of time during the exam in which the signal data is representative of the user performing the relevant activities of the exam and may generate one or more annotations associated with the context window, such as describing a predicted rating on the task, predicting a subjective input from the user on the task, or other such information. To do so, the wearable device may process the sensor data through a machine learning algorithm trained using previous clinical exam data and other VME data that includes established or truth labels as set by clinicians or wearer input directly. In some examples, the sensor data may be segmented and subsequently processed to generate one or more annotations describing contexts, performance, predicted ratings, and other such information. The sensor data may be stored at the remote server or at a data storage device. The processing and generation of machine-learning algorithm annotations may be performed at the remote server or at the wearable device, though additional computing resources available at the remote server may result in faster processing and generation of annotations. In some examples, the output of the machine-learning algorithm can also be used to train, calibrate, or otherwise adjust the operation of the machine learning algorithm, or to train a new machine-learning algorithm for generating further refined annotations.

As an extension of the particular example, the wearer may perform one or more tasks similar or identical to a task performed as part of a VME. In this example, the wearer may, for example, sit with their hands in their lap and still while watching television or in some other situation. Though they may not be consciously choosing to perform the task of the VME, the machine-learning algorithm may identify, from sensor data, that the wearer is performing the task, or a task similar to the prescribed task and may provide annotations to identify the task and provide a rating, context, or other such information. In this way, the machine-learning algorithm, which may be one or more algorithms performing discrete tasks (e.g., identifying a task similar to a VME task by a first algorithm and generating an annotation describing a context or performance on the task with a second algorithm) enables further data gathering and provides a more complete snapshot of the wearer's symptoms and disease progression.

The systems and methods provided herein enable better tracking of disease progression and data gathering related to clinical trials and diagnoses. Data gathered during a visit to a clinic provide only a single snapshot of the progress of a disease, a treatment, or other such information, and such a snapshot may only be compared against a relatively infrequent additional snapshot from a further visit. Using the systems and techniques described herein, clinical data may be used to train machine-learning algorithms, including data from VMEs, and used to identify and annotate sensor data gathered in between clinical visits, and potentially gathered continuously over a treatment span to provide a more complete view of the progress of a treatment or other such medical care. Rather than taxing a medical system with clinical visits of a high frequency, the systems and methods described herein enable data to be gathered and annotated such that a clinical professional can review annotations and sensor data at regular intervals and have a clear understanding of the progress and day-to-day impact of a treatment or progression of a disease.

This illustrative example is given to introduce the reader to the general subject matter discussed herein, and the disclosure is not limited to this example. The following sections describe various additional non-limiting examples of techniques relating to automatic activity identification and annotation of tasks performed by a wearer of an example wearable sensor device collected during VMEs as well as throughout a typical day in a free-living setting.

### I. EXAMPLE STUDY USING WEARABLE DEVICE AND TECHNIQUES DESCRIBED HEREIN

### A. Introduction of Example Study

As described herein, sensor-based remote monitoring may help health care professionals better track disease progression such as in Parkinson's disease (PD), and measure users' response to putative disease-modifying therapeutic interventions. To be successful, the remotely-collected measurements should be valid, reliable and sensitive to change, and people with PD must engage with the technology.

The wearable device described herein may be used to implement a smartwatch-based active assessment that enables unsupervised measurement of motor signs of PD. In an example study, 388 study users with early-stage PD (Personalized Parkinson Project, 64% men, average age 63 years) wore a smartwatch for a median of 390 days, allowing for continuous passive monitoring. Users performed unsupervised motor tasks both in the clinic (once) and remotely (twice weekly for one year). Dropout rate was 2% at the end of follow-up. Median wear-time was 21.1 hours/day, and 59% of per-protocol remote assessments were completed.

In the example study, in-clinic performance of the virtual exam verified that most users correctly followed watch-based instructions. Analytical validation was established for in-clinic measurements, which showed moderate-to-strong correlations with consensus Movement Disorder Society - Unified Parkinson's Disease Rating Scale (MDS-UPDRS) Part III ratings for rest tremor (ρ=0.70), bradykinesia (ρ=-0.62), and gait (ρ=-0.46). Test-retest reliability of remote measurements, aggregated monthly, was good-to-excellent (ICC: 0.75 - 0.96). Remote measurements were sensitive to the known effects of dopaminergic medication (on vs off Cohen's d: 0.19 - 0.54). Of note, in-clinic assessments often did not reflect the users' typical status at home.

In the example study, the feasibility of using smartwatch-based unsupervised active tests was demonstrated, and established the analytical validity of associated digital measurements. Weekly measurements can create a more complete picture of user functioning by providing a real-life distribution of disease severity, as it fluctuates over time. Sensitivity to medication-induced change, together with the improvement in test-retest reliability from temporal aggregation implies that these methods could help reduce sample sizes needed to demonstrate a response to therapeutic intervention or disease progression.

The smartwatch-based Parkinson's Disease Virtual Motor Exam (PD-VME) can be deployed to remotely measure the severity of tremor, bradykinesia and gait impairment, via a self-guided active assessment. In the example study, the feasibility of use and quality of data collected by the system was evaluated, and report on the reliability, validity, and sensitivity to change of a set of digital measures derived from the PD-VME during a multi-year deployment in the Personalized Parkinson Project (PPP) was formed.

### B. Study Design

Data were collected as part of the ongoing Personalized Parkinson Project (PPP), a prospective, longitudinal, single-center study of 520 people with early-stage Parkinson's disease - diagnosed within the last 5 years. Study users wore a smartwatch such as the wearable device described herein for up to 23 hours/day for the 3-year duration of the study, which passively collects raw sensor data from IMU, gyroscope, photoplethysmography, skin conductance sensors, and/or any other suitable sensor. All sensor data collected in this study used a wrist-worn wearable device.

Sensor data was collected during the yearly in-clinic MDS-UPDRS Part III motor exams. These were conducted in both the on and off states, after overnight withdrawal of dopaminergic medication (at least 12 hours after the last intake). Exams were video-recorded for quality controls and offline consensus scoring. Set 1 (N=198 users) was selected for video-based consensus scoring by matching age, gender and MDS-UPDRS III score to be representative of the overall PPP study. Two assessors independently scored videos of the exams. When difficulties in rating MDS-UPDRS Part III tasks arose due to poor video quality, assessors provided scores only when confident in their assessment. MDS-UPDRS Part III consensus scores were computed as the median of the in-person rating and both video ratings.

Initially, users were offered the opportunity to enroll in a substudy, which asks them to perform an active assessment (Parkinson's Disease Virtual Motor Exam, PD-VME) in the clinic and in remote, unsupervised settings. The PD-VME was deployed fully remotely, using digital instructions and an over-the-air firmware update to the watches of consented users. 370 users enrolled in the substudy (Set 2).

The smartwatch guides users through the series of structured motor tasks comprising the PD-VME. It also allows users on symptomatic medication to log the timing of their medication intake, which included a user-facing UI of the PD-VME.

Each week, users were asked to perform the PD-VME twice on the same day, at two predefined times: first in the off state (selected as a time when they typically experienced their worst motor function), and then in the on state (at a time when they typically experienced good motor function later in the day). Users not taking medication were instructed to complete the PD-VME twice, one hour apart. The helpdesk at the site monitored wear-time and PD-VME completion and reached out to users if more than three consecutive weekly assessments were missed.

Later on, users enrolled in the PD-VME substudy were asked to perform the PD-VME during their in-clinic visit (in the same manner as they did remotely), while the assessor observed its execution without providing feedback or any additional instructions. The in-clinic PD-VME is performed within 1 hour after completion of the MDS-UPDRS part III off state exam, and before dopaminergic medication intake.

### C. Design of Virtual Motor Exam

The PD-VME system, including user-facing training materials, user interface, task choice and digital measures, was developed using a user-centric approach. The PD-VME may include eight tasks designed to assess various domains of motor signs: rest and postural tremor, upper extremity bradykinesia through finger tapping, pronation-supination and repeated hand opening and closing, lower-extremity bradykinesia through foot stomping, gait and postural sway. A PD-VME user interface for the four tasks was used. Selection of targeted signs was informed by research on meaningful aspects of health in PD: tremor, bradykinesia and gait were identified as three of the top four symptoms that people with PD most want to improve. A user panel of PPP users was involved throughout the design process to assess and improve the usability of the system.

During execution of PD-VME tasks, tri-axial accelerometer and gyroscope data was collected at a sample rate of 200 Hz. For each task, an initial list of concepts of interest were identified (e.g., tremor severity, quality of gait). For each concept, digital signal processing was implemented to convert the raw sensor data into 11 exploratory outcome measures (e.g., tremor acceleration, arm-swing magnitude).

### D. Evaluation of Digital Measures from PD-VME

User engagement with the PD-VME, measured as the fraction of users who performed at least one complete exam in a given week, was evaluated over the course of 70 weeks. The ability of the users to perform the PD-VME correctly without having received in-person instructions was assessed using the assessor observations from the in-clinic PD-VME.

The analytical validity, reliability, and sensitivity to change of digital measurements from the PD-VME was evaluated. First, the analytical validity of measures, collected during the in-clinic MDS-UPDRS, was assessed using the Spearman correlation coefficient of the measure against the consensus of three independent MDS-UPDRS Part III clinical ratings. Second, the test-retest reliability in the home setting was evaluated by computing the intra-class correlation between monthly means across subsequent months for months with no missing PD-VME. Finally, the sensitivity to change was assessed by testing the ability of the remote measurements to distinguish between the off and the on states for the subset of users in Set 2 who are on dopaminergic medication. An unsupervised PD-VME exam is determined to be in the off state if it occurred at the pre-scheduled off time and at least 6 hours after a medication tag. Similarly, an exam is determined to be in the on state if it occurred at the pre-scheduled on time and between 0.5 and 4 hours after a medication tag. Two measures were used to assess the magnitude of change: mean difference (and associated 95% confidence interval) and Cohen's d. Users taking dopamine agonists were not included in the on-off comparison because of their prolonged effect.

For each task, one outcome measure is shown in the results, selected on the basis of its high performance across all three aspects (analytical validity, test-retest reliability and sensitivity to change) for inclusion in the results.

### E. Comparison of In-clinic and Remotely Collected PD-VME Measurements

To characterize the extent to which measures obtained from clinic-based physical exams (off) reflected users' signs in the remote setting (off), the distributions of users' in-clinic and remote PD-VME outcomes (completed within 90 days of the clinic visit) were compared. A subset of N=194 users from Set 2 who performed the PD-VME in-clinic was included in this analysis.

Statistical analyses were generated using the Python programming language, using the SciPy, Matplotlib, and seaborn libraries. In all numerical results that follow, point estimates are followed by 95% confidence intervals in square brackets. Confidence intervals were calculated using the bootstrap method with 1000 resampling iterations.

### F. Results

### 1. Engagement

Median smartwatch wear time across all PPP users (N=520) was 22.1 hours/day, with a median follow-up period of 390 days. Variations in follow-up duration were due largely to the N=126 who have not completed the study, and loss-to-follow-up is only 2%. Users in Set 2 completed 22,668 PD-VMEs, corresponding to 59% of per-protocol test sessions during the 70-week follow-up period. In the first week, 80% of users had at least 1 PD-VME, and 40% had completed one PD-VME in week 52.

### 2. Useability

Users' ability to perform the PD-VME was assessed during the in-clinic visit. Users were able to complete the tasks in the exam (100% for tremor and upper-extremity bradykinesia and 98.5% for gait). Major protocol deviations were recorded as follows: users did not place their hands on their lap during rest tremor tasks (8.2% of cases), users performed the arm-twist using both arms (3.1% of cases), and users either walked with their arms crossed across their chest (in 3.1% of cases) or sat down repeatedly (6.8% of cases) during the gait task.

### 3. Rest Tremor

Among three measurements that were considered for measuring tremor severity, lateral tremor acceleration measurement was presented here because it showed the strongest correlation to in-clinic MDS-UPDRS ratings, and the strongest ability to separate on from off state measurements.

The Spearman rank correlation between the median lateral acceleration during the rest tremor task and expert consensus rating of MDS-UPDRS task 3.17 was 0.70 [0.61, 0.78], N=138. For 56 users, video quality was insufficient to ensure high confidence consensus ratings

Wrist acceleration signals intuitively map to the clinical observations during the MDS-UPDRS.Next, the sensitivity to on-off changes of the rest-tremor acceleration measurement was assessed. A small effect (Cohen's d of 0.2) was observed comparing the on and off state. The mean difference in the measure was 0.10 [0.06, 0.14].

Intra-class correlation (ICC) of 0.71 [0.58-0.81] week-on-week (N=208), and ICC of 0.90 [0.84-0.94] m.s-2 for monthly averaged measures (N=139) was identified for test-retest reliability.

The in-clinic PD-VME measure was between the 25th and the 75th percentiles of the remote PD-VME measures for 41% of the users.

### 4. Upper-extremity bradykinesia

Among the four measurements that were considered for measuring upper-extremity bradykinesia severity, no single measure showed both strong correlation to in-clinic MDS-UPDRS ratings, and a strong ability to separate on from off state measurements. Therefore, results are included below for both the arm-twist amplitude, and the arm-twist rate.

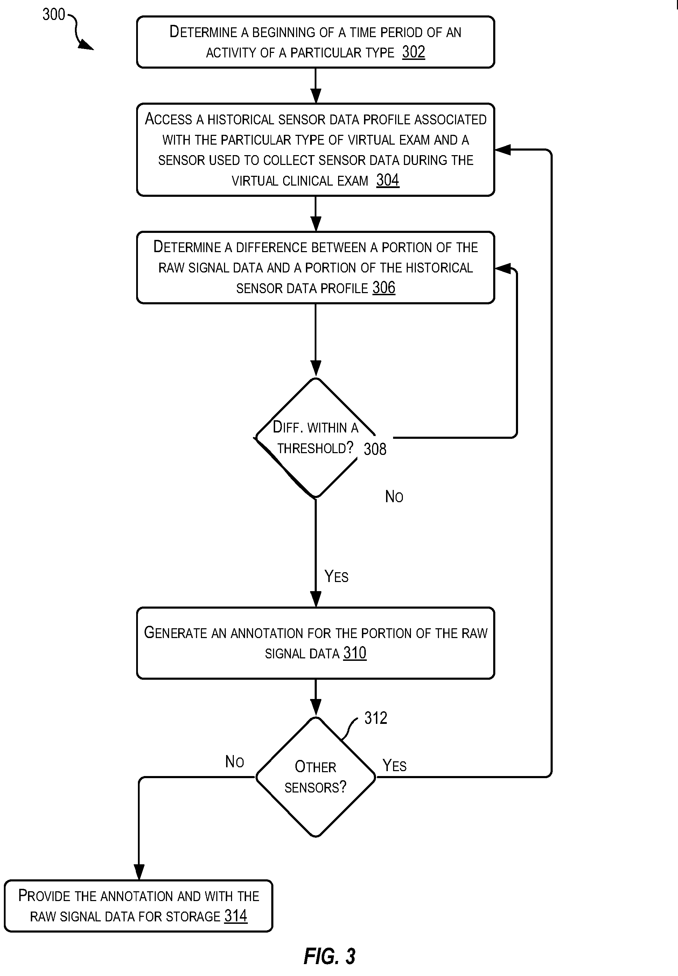

The highest correlation with expert consensus rating of MDS-UPDRS task 3.6 was observed for the arm twist amplitude measure, with ρ = -0.62 [-0.73, -0.50], N=159 (Fig. 3.A). However, the effect of medication state (Cohen's d of -0.07) was very small (Fig. 3.C).35 The mean on-off difference in the measure was -0.9 [0.0, -1.6] degrees. Test-retest ICC was 0.71 [0.59-0.80] week-on-week (N=208) and 0.89 [0.84-0.94] for monthly-averaged measures (N=136). The in-clinic PD-VME measure was between the 25th and the 75th percentiles of the remote PD-VME measures for 45% of the users.

The assessors observed during the in-clinic PD-VME exam that some users mainly focused on the speed of the arm-twist movement rather than the amplitude. Therefore, sensor-based measures of the rate of arm-twist and the combination of rate and amplitude were investigated as well. Correlations to the consensus MDS-UPDRS ratings of ρ = 0.06 [-0.25, +0.13] for arm-twist rate, and ρ = -0.42 [-0.55, -0.28] for the product of rate and amplitude were observed. Both metrics showed significant change in on and off: Cohen's d of -0.22 and mean change of -0.16 [-0.13, -0.20] s-1 for arm-twist rate, and Cohen's d of -0.26 and mean change of -8 [-6, -10] degrees/s for the combination.

### 5. Arm Swing During Gait

Among the three measurements that were considered for measuring gait impairment, arm swing acceleration was selected. While it was not the best outcome measure across any of the criteria, it showed solid performance across all of them.

The Spearman rank correlation between the arm swing acceleration during the gait task and expert consensus rating of MDS-UPDRS task 3.10 was ρ = -0.46 [-0.57, -0.31], N=164. A small effect (Cohen's d of 0.44) was observed comparing the on and off state. The mean difference in the measure was -0.8 [-1.2, -0.5] m.s-2. Test-retest ICC was 0.43 [0.30-0.56] week-on-week (N=210), and 0.75 [0.66-0.84] for monthly-averaged measures (N=139). The in-clinic PD-VME measure was between the 25th and the 75th percentiles of the remote PD-VME measures for 39% of the users.

### G. Discussion

In some examples, people with PD may engage with and are able to use the PD-virtual motor exam, and the quality of data collected during a study environment may be high enough to enable evaluation of the analytical validity, reliability, and sensitivity to change of digital measures built from the system.

In some examples, a digital exam solution may be useful when people with PD engage with it regularly. For example, robust levels of engagement, both in terms of overall wear time (>21 hours/day) and engagement with the active assessment, may be performed over one or more years when assayed on a weekly basis. In some examples, combining active assessments with passive monitoring on wearable device form-factors may have the potential to yield substantial quantities of high quality data. For studies assessing longitudinal progression, even higher engagement may be obtained by requiring a set of weekly unsupervised tests for a limited duration at baseline and again at the end of the follow-up period.

In some examples, moderate-to-strong correlation may be shown between in-clinic MDS-UPDRS Part III measurements and consensus clinical ratings for rest tremor, bradykinesia, and arm swing during gait, which may provide analytical validation of the individual measurements. While the moderate-to-strong correlations with MDS-UPDRS scores may establish that the measurements are working as intended, engineering for perfect correlation may recreate an imperfect scoring system, and may wash out the potential for increased sensitivity of sensor-based measurements. One key reason for making a shift towards digital assessments is that clinical scores may remain subjective in nature, and may use a low resolution, ordinal scoring system. The criteria for transitioning between different scores may leave room for subjective interpretation, and may cause considerable variability between and within raters in daily practice.

This is exemplified by the results shown for the upper-extremity bradykinesia measure, in which it has been found that the measure most correlated with in-clinic MDS-UPDRS ratings - amplitude of arm-twist - is not the one that is most sensitive to change from dopaminergic medication. It is possible that while the experts are instructed to evaluate "speed, amplitude, hesitations, halts and decrementing amplitude", they may focus mostly on amplitude. Similarly, it could be observed that a gradient of tremor measurements, both in-clinic and remotely, even within the group of users who are rated as a 0 on the MDS-UPDRS 3.15 or 3.17. This may suggest that some amount of tremor could be present, both in the clinic and at-home, even before it becomes apparent to the human eye. Indeed, it is generally a well-accepted phenomenon that tremors are easier felt or even heard (using a stethoscope) than observed by an examiner. This reinforces the need for objective sensor-based measures, and the need to evaluate these measures based on their ability to detect clinically meaningful changes rather than simply reproducing subjective clinical exams.

In people with PD, dopaminergic medication can considerably improve severity of motor signs over short time frames. This "on-off" difference is well-accepted as a clinically meaningful change, and when coupled with wearable sensors and user-reported tagging of daily medication regimen, creates multiple "natural experiments" in the course of users' daily lives. These may allow testing of the clinical validity of the PD-VME measures as pharmacodynamic/response biomarkers for people with PD in the remote setting. Indeed, digital measures for tremor, upper-extremity bradykinesia and gait may be able to detect significant change in users' motor signs before and after medication intake.

For clinical trials aiming to show disease modification, measurements that provide reliable estimates of a subject's disease state can increase statistical power, and reduce the required sample size or trial duration. However, measuring long-term progression using infrequent measurements is difficult, because motor and non-motor signs of PD can markedly fluctuate from moment to moment, depending on factors such as the timing of medication intake or the presence of external stressors. The increased test-retest reliability of the monthly aggregated measures may suggest that collecting outcome measures remotely and at an increased frequency increases their reliability, and has the potential to measure progression of the average motor sign severity.

Users that engage robustly with the PD-VME may be able to conduct assessments of motor function to yield data of a sufficient quality to generate digital measurements of motor signs, test their analytical validity, and assess their sensitivity to change in medication status. The system may allow for an increased frequency of data collection, enabling monthly aggregation of measurements, leading to increased test-retest reliability. In turn, high reliability suggests that these measures have potential as digital biomarkers of progression.

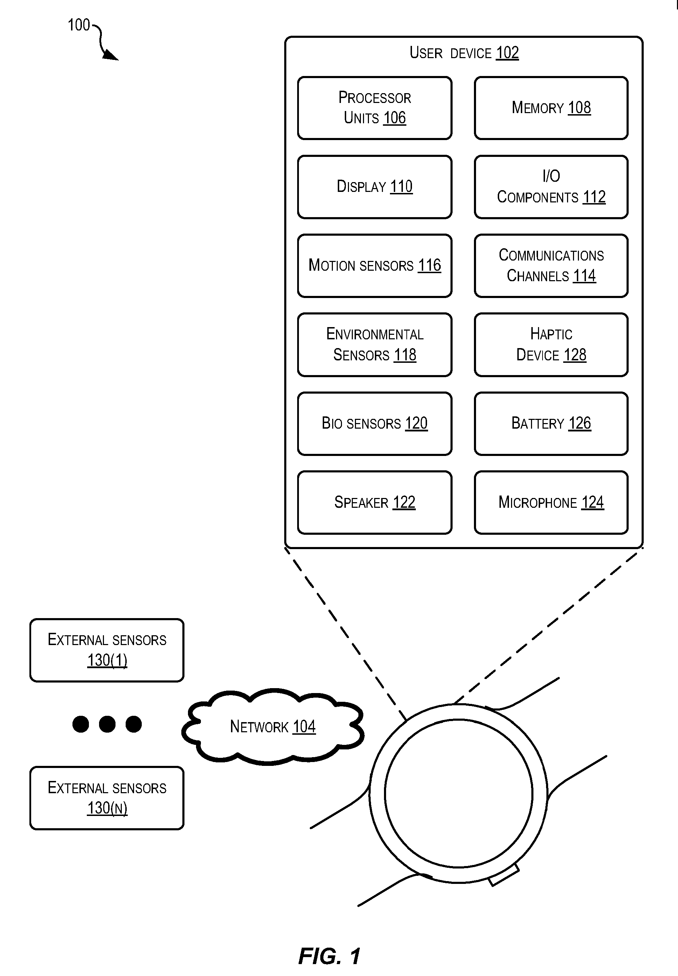

Turning now to the figures, FIG. 1 illustrates an example system including a user device for use in implementing techniques related to activity identification and automatic annotating of sensor data from a wearable sensor device, according to at least one example. The system 100 includes a user device 102 such as wearable sensor device that may communicate with various other devices and systems via one or more networks 104.

Examples described herein may take the form of, be incorporated in, or operate with a suitable wearable electronic device such as, for example, a device that may be worn on a user's wrist and secured thereto by a band, a device worn around the user's neck or chest, etc.. The device may have a variety of functions, including, but not limited to: keeping time; monitoring a user's physiological signals and providing health-related information based at least in part on those signals; communicating (in a wired or wireless fashion) with other electronic devices, which may be different types of devices having different functionalities; providing alerts to a user, which may include audio, haptic, visual, and/or other sensory output, any or all of which may be synchronized with one another; visually depicting data on a display; gathering data from one or more sensors that may be used to initiate, control, or modify operations of the device; determining a location of a touch on a surface of the device and/or an amount of force exerted on the device, and using either or both as input; accepting voice input to control one or more functions; accepting tactile input to control one or more functions; and so on. Though examples are shown and described herein with reference to a wearable sensor device worn on a user's wrist, other wearable sensors are envisioned such as sensor devices in rings, patches, clothing, and other such wearable sensor devices and systems.

As shown in FIG. 1, the user device 102 includes one or more processor units 106 that are configured to access a memory 108 having instructions stored thereon. The processor units 106 of FIG. 1 may be implemented as any electronic device capable of processing, receiving, or transmitting data or instructions. For example, the processor units 106 may include one or more of: a microprocessor, a central processing unit (CPU), an application-specific integrated circuit (ASIC), a digital signal processor (DSP), or combinations of such devices. As described herein, the term "processor" is meant to encompass a single processor or processing unit, multiple processors, multiple processing units, or other suitably configured computing element or elements.

The memory 108 may include removable and/or non-removable elements, both of which are examples of non-transitory computer-readable storage media. For example, non-transitory computer-readable storage media may include volatile or non-volatile, removable or non-removable media implemented in any method or technology for storage of information such as computer-readable instructions, data structures, program modules, or other data. The memory 108 is an example of non-transitory computer storage media. Additional types of computer storage media that may be present in the user device 102 may include, but are not limited to, phase-change RAM (PRAM), static random-access memory (SRAM), dynamic random-access memory (DRAM), random-access memory (RAM), read-only memory (ROM), electrically erasable programmable read-only memory (EEPROM), flash memory or other memory technology, compact disc read-only memory (CD-ROM), digital video disc (DVD) or other optical storage, magnetic cassettes, magnetic tape, magnetic disk storage or other magnetic storage devices, or any other medium that can be used to store the desired information and that can be accessed by the user device 102. Combinations of any of the above should also be included within the scope of non-transitory computer-readable storage media. Alternatively, computer-readable communication media may include computer-readable instructions, program modules, or other data transmitted within a data signal, such as a carrier wave, or other transmission. However, as used herein, computer-readable storage media does not include computer-readable communication media.

In addition to storing computer-executable instructions, the memory 108 may be configured to store raw sensor data and annotations associated with the sensor data. In some examples, the annotations may be produced by the user device 102 by executing one or more instructions stored on the memory 108, such as instructions for processing, via a machine-learning algorithm, sensor data to produce annotations associated with the sensor data.. Machine-learning techniques may be applied based on training data sets from clinical data or other data established as truth, such as from data entered by clinicians associated with a VME. The stored sensor data, annotations, or other such data may be stored at the memory 108 or at a remote server, for example communicated across network 104.

The instructions or computer programs may be configured to perform one or more of the operations or functions described with respect to the user device 102. For example, the instructions may be configured to control or coordinate the operation of the various components of the device. Such components include, but are not limited to, display 110, one or more input/output (I/O) components 112, one or more communication channels 114, one or more motion sensors 116, one or more environmental sensors 118, one or more bio sensors 120, a speaker 122, microphone 124, a battery 126, and/or one or more haptic devices 128.

The display 110 may be configured to display information via one or more graphical user interfaces and may also function as a input component, e.g., as a touchscreen. Messages relating to the execution of exams may be presented at the display 110 using the processor units 106.

The I/O components 112 may include a touchscreen display, as described, and may also include one or more physical buttons, knobs, and the like disposed at any suitable location with respect to a bezel of the user device 102. In some examples, the I/O components 112 may be located on a band of the user device 102.

The communication channels 114 may include one or more antennas and/or one or more network radios to enable communication between the user device 102 and other electronic devices such as one or more other external sensors 130, other electronic devices such as a smartphone or tablet, other wearable electronic devices, external computing systems such as a desktop computer or network-connected server. In some examples, the communication channels 114 may enable the user device 102 to pair with a primary device such as a smartphone. The pairing may be via Bluetooth or Bluetooth Low Energy (BLE), near-field communication (NFC), or other suitable network protocol, and may enable some persistent data sharing. For example, data from the user device 102 may be streamed and/or shared periodically with the smartphone, and the smartphone may process the data and/or share with a server. In some examples, the user device 102 may be configured to communicate directly with the server via any suitable network 104, e.g., the Internet, a cellular network, etc.

The sensors of the user device 102 may be generally organized into three categories including motion sensors 116, environmental sensors 118, and bio sensors 120, though other sensors or different types or categories of sensors may be included in the user device 102. As described herein, reference to "a sensor" or "sensors" may include one or more sensors from any one and/or more than one of the three categories of sensors including other sensors that may not fit into only one of the categories. In some examples, the sensors may be implemented as hardware elements and/or in software.

Generally, the motion sensors 116 may be configured to measure acceleration forces and rotational forces along three axes. Examples of motion sensors include accelerometers, gravity sensors, gyroscopes, rotational vector sensors, significant motion sensors, step counter sensor, Global Positioning System (GPS) sensors, and/or any other suitable sensors. Motion sensors may be useful for monitoring device movement, such as tilt, shake, rotation, or swing. The movement may be a reflection of direct user input (for example, a user steering a car in a game or a user controlling a ball in a game), but it can also be a reflection of the physical environment in which the device is sitting (for example, moving with a driver in a car). In the first case, the motion sensors may monitor motion relative to the device's frame of reference or your application's frame of reference; in the second case the motion sensors may monitor motion relative to the world's frame of reference. Motion sensors by themselves are not typically used to monitor device position, but they can be used with other sensors, such as the geomagnetic field sensor, to determine a device's position relative to the world's frame of reference. The motion sensors 116 may return multi-dimensional arrays of sensor values for each event when the sensor is active. For example, during a single sensor event the accelerometer may return acceleration force data for the three coordinate axes, and the gyroscope may return rate of rotation data for the three coordinate axes.

Generally, the environmental sensors 118 may be configured to measure environmental parameters such as temperature and pressure, illumination, and humidity. The environmental sensors 118 may also be configured to measure physical position of the device. Examples of environmental sensors 118 may include barometers, photometers, thermometers, orientation sensors, magnetometers, Global Positioning System (GPS) sensors, and any other suitable sensor. The environmental sensors 118 may be used to monitor relative ambient humidity, illuminance, ambient pressure, and ambient temperature near the user device 102. In some examples, the environmental sensors 118 may return a multi-dimensional array of sensor values for each sensor event or may return a single sensor value for each data event. For example, the temperature in °C or the pressure in hPa. Also, unlike motion sensors 116 and bio sensors 120, which may require high-pass or low-pass filtering, the environmental sensors 118 may not typically require any data filtering or data processing.

The environmental sensors 118 may also be useful for determining a device's physical position in the world's frame of reference. For example, a geomagnetic field sensor may be used in combination with an accelerometer to determine the user device's 102 position relative to the magnetic north pole. These sensors may also be used to determine the user device's 102 orientation in some of frame of reference (e.g., within a software application). The geomagnetic field sensor and accelerometer may return multi-dimensional arrays of sensor values for each sensor event. For example, the geomagnetic field sensor may provide geomagnetic field strength values for each of the three coordinate axes during a single sensor event. Likewise, the accelerometer sensor may measure the acceleration applied to the user device 102 during a sensor event. The proximity sensor may provide a single value for each sensor event.

Generally, the bio sensors 120 may be configured to measure biometric signals of a wearer of the user device 102 such as, for example, heartrate, blood oxygen levels, perspiration, skin temperature, etc. Examples of bio sensors 120 may include a hear rate sensor (e.g., photoplethysmography (PPG) sensor, electrocardiogram (ECG) sensor, electroencephalography (EEG) sensor, etc.), pulse oximeter, moisture sensor, thermometer, and any other suitable sensor. The bio sensors 120 may return multi-dimensional arrays of sensor values and/or may return single values, depending on the sensor.

The acoustical elements, e.g., the speaker 122 and the microphone 124 may share a port in housing of the user device 102 or may include dedicated ports. The speaker 122 may include drive electronics or circuitry and may be configured to produce an audible sound or acoustic signal in response to a command or input. Similarly, the microphone 124 may also include drive electronics or circuitry and is configured to receive an audible sound or acoustic signal in response to a command or input. The speaker 122 and the microphone 124 may be acoustically coupled to a port or opening in the case that allows acoustic energy to pass, but may prevent the ingress of liquid and other debris.

The battery 126 may include any suitable device to provide power to the user device 102. In some examples, the battery 126 may be rechargeable or may be single use. In some examples, the battery 126 may be configured for contactless (e.g., over the air) charging or near-field charging.

The haptic device 128 may be configured to provide haptic feedback to a wearer of the user device 102. For example, alerts, instructions, and the like may be conveyed to the wearer using the speaker 122, the display 110, and/or the haptic device 128.

The external sensors 130(1)-130(n) may be any suitable sensor such as the motion sensors 116, environmental sensors 118, and/or the bio sensors 120 embodied in any suitable device. For example, the external sensors 130 may be incorporated into other user devices, which may be single or multi-purpose. For example, a heartrate sensor may be incorporated into a chest band that is used to capture heartrate data at the same time as the user device 102 captures sensor data. In other examples, position sensors may be incorporated into devices and worn at different locations on a human user. In this example, the position sensors may be used to track positional location of body parts (e.g., hands, arms, legs, feet, head, torso, etc.). Any of the sensor data obtained from the external sensors 130 may be used to implement the techniques described herein.

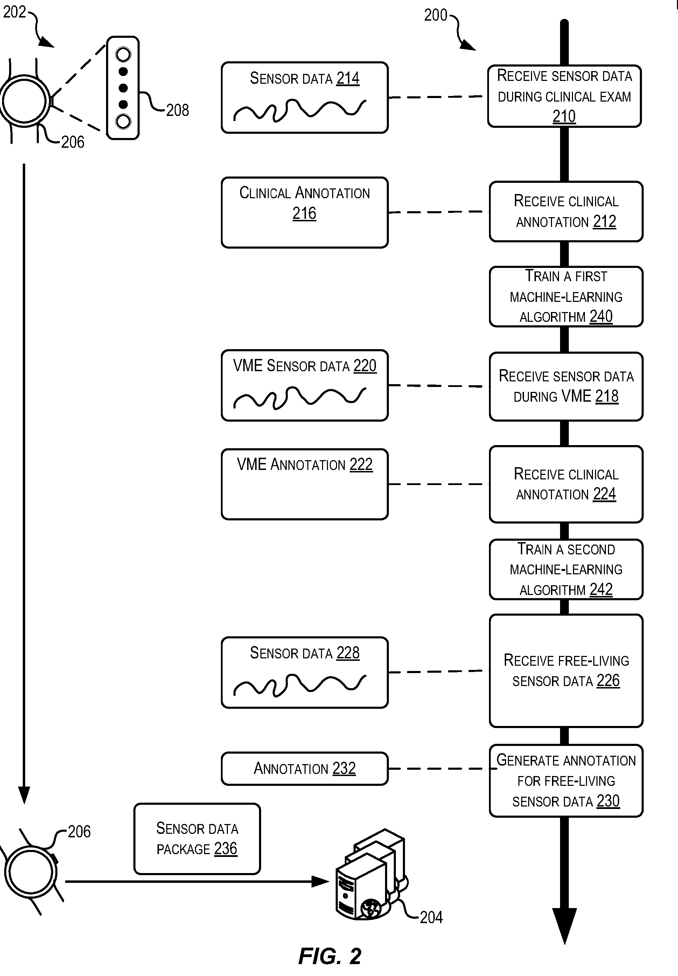

FIG. 2 illustrates a system 202 and a corresponding flowchart illustrating a process 200 for identifying activities in and automatically annotating sensor data of wearable sensor devices, according to at least one example. The system 202 includes a service provider 204 and a user device 206. FIG. 2 illustrates certain operations taken by the user device 206 as it relates to identifying and annotating sensor data. The user device 206 is an example of the user device 102 introduced previously.

As described in further detail herein, the service provider 204 may be any suitable computing device (e.g., personal computer, handheld device, server computer, server cluster, virtual computer) configured to execute computer-executable instructions to perform operations such as those described herein. The computing devices may be remote from the user device 206. The user device 206, as described herein, is any suitable portable electronic device (e.g., wearable device, handheld device, implantable device) configured to execute computer-executable instructions to perform operations such as those described herein. The user device 206 includes one or more sensors 208. The sensors 208 are examples of the sensors 116-120 described herein.

The service provider 204 and the user device 206 may be in network communication via any suitable network such as the Internet, a cellular network, and the like. In some examples, the user device 206 may be intermittently in network communication with the service provider 204. For example, the network communications may be enabled to transfer data (e.g., raw sensor data, annotation data, adjustment information, user input data) which can be used by the service provider 204 for identifying activities and generating annotations identifying the activities and adding annotations describing one or more contexts or aspects of the activities. In some examples, the processing may be performed on the user device 206 or on a primary device. The primary device may be a computing device, or include a computing device in communication with the user device 206 and that may, in some examples perform some, or all of a portion of data processing. In this manner, the primary device may reduce a computational load on the user device 206 which may in turn enable the use of less sophisticated computing devices and systems built into the user device 206. In some examples, the user device 206 is in network communication with the service provider 204 via a primary device. For example, the user device 206, as illustrated, may be a wearable device such as a watch. In this example, the primary device may be a smartphone that connects to the wearable device via a first network connection (e.g., Bluetooth) and connects to the service provider 204 via a second network connection (e.g., cellular). In some examples, however, the user device 206 may include suitable components to enable the user device 206 to communicate directly with the service provider 204.

The process 200 illustrated in FIG. 2 provides an overview of how the system 202 may be employed to automatically identify and annotate activities within sensor data. The process 200 may begin at block 210 by the user device 206 receiving sensor data during a clinical exam. Though the process is described with the user device 206 receiving data and information, in some examples, the service provider may receive the information from one or more sources, such as from the user device 206, a primary device, a clinical device, and other such locations. The sensor data may be generated by the user device 206 during or after clinical exam in a clinical setting where one or more tasks have been conducted. The sensor data may include information and obtained by a sensor 208(1) (e.g., one of the sensors 208). In some examples, the sensor data 214 may have been collected during the exam identified a clinical annotation 216 accessed at block 212. Blocks 201 and 21 may be performed while a user is within a clinical environment. In some examples, the sensor data 214 may be processed by the sensor that generates the sensor data 214 (e.g., filters, digitizes, packetizes, etc.). In some examples, the sensors 208 provide the sensor data 214 without any processing. Logic on the user device 206 may control the operation of the sensors 208 as it relates to data collection during the exam. All of the sensors 208 may be time-aligned because they are all on the same device (e.g., the user device 206) and thereby aligned with the same internal clock (e.g., a clock of the user device 206).

At block 212, the user device 206 receives clinical annotations 216 that may indicate characteristics of a VME, such as a type of VME, a task associated with the type of VME, user- or system-provided timestamps identifying a beginning and an end of the exam, user-provided feedback about the exam, and other information about the exam including a clinician rating on the performance of the task, and other such clinical exam annotations. In some examples, the user device 206 accesses the clinical annotations 216 information from a memory of the user device 206 or from a clinical device.

At block 240, a first machine-learning algorithm is trained using the sensor data 214 and the clinical annotations 216. The first machine-learning algorithm is trained based on annotation placed by clinicians or during and in response to the clinical exam, the clinical annotations 216 being associated with particular portions of the sensor data 214. The first machine-learning model may therefore be a rough model capable of producing annotations similar to those produced in the clinical annotations 216.

At block 218, the user device 206 receives sensor data 220 during a VME. The sensor data 220 may include data similar to sensor data 214, including data from sensors 208 of the user device 206 while the user performs a VME. The VME may be clearly identified with tags that mark a start and an end time of the VME within the sensor data. The VME may be performed by the user following instructions displayed on the display of the user device 206. The sensor data 220 from the VME may be conveyed to a clinician for analysis during or after performance of the task for evaluation of the performance.

At block 224, the user device 206 receives VME annotations 222 that may indicate the start and end time of the task, the type of task performed, and other such information related to the performance of the VME. The VME annotations 222 may include corroborative data from additional sensors of other devices, such as sensors indicating a stationary position of a user during a stationary task or location data indicating motion during a moving task. The VME annotation 222 may also include annotations that may be added by a clinician such as to indicate general performance, provide rating information, or otherwise. The VME annotation 222 may also include user-input information, such as a rating from a user for a level of pain or difficulty completing the task. The user device 206, may prompt the user to input such information following completion of the VME. The user device 206 may prompt the user with various questions relating to performance, difficulty, whether the user has taken a medication on time and recently, and other such data. In some examples, the questions from the user device 206 may include both objective and subjective information, including as described above. The user device 206 may pose questions targeting similar information or responses in different phrasing, to elicit multiple responses from the user and thereby ensure consistency or provide additional data points that may be used to average potentially volatile subjective data.

In some examples, the VME annotations 222 may be generated by the first machine-learning algorithm, trained at block 240. The first machine-learning algorithm may produce predicted ratings for VME annotations, such as a predicted score for a particular scorable task. The first-machine learning algorithm may also produce predicted subjective annotations, for example identifying similarities between when a user input information describing a level of pain during a task and identifying similarities between the sensor data and thereby determining or predicting a similar subjective input from the user. The similarities may be identified as a score, and may be used to select an annotation for application with the VME sensor data 220 when the similarity score exceeds a predetermined threshold.

In some examples, the annotations produced by the first machine-learning algorithm may be confirmed or corroborated by other sensor devices, user inputs, or clinician inputs. For example, the user device 206 may prompt the user to verify a level of pain or difficulty predicted by the first machine-learning algorithm. The user device 206 may also gather corroborative data from additional sensors, for example to confirm a level of tremor or shaking or to confirm user motion during the task. The annotation may likewise be confirmed by a clinician in some examples. In one instance, a VME may be performed during a virtual care session or a clinician may be able to view a recorded video of the VME, and the clinician may be able to confirm one or more annotations, for example with predicted performance scores on the VME task or other notes.

At block 242, a second machine-learning algorithm may be trained using the VME sensor data 220, sensor data 214, clinical annotation 216, and VME annotation 222. The second machine-learning algorithm may be similar or identical to the first machine-learning algorithm, and with the additional training data from the VME sensor data 220 and the VME annotations 222, the second machine-learning algorithm may produce additional annotations, more accurate annotations, and further be capable of identifying activities associated with the VME, or other tasks, without input from the user indicating the start of a task.

At block 226, the user device 206 gathers free-living sensor data 228. The free-living sensor data 228 includes raw sensor data gathered as the user wears the user device 206 throughout an extended period of time beyond a time period for a clinical or virtual exam. In some examples, the free-living sensor data 228 may include continuous sensor data corresponding to full days, weeks, or months of data gathered as the user goes about their typical daily routines.

At block 230, the second machine-learning algorithm may generate annotations 232 corresponding to the free-living sensor data 228. The annotations 232 may be generated at the user device 206 rather than after sending the sensor data to a remote server. In this manner, the appended sensor data including the annotations 232 may be sent from the user device 206 as described below. The annotations 232 may be more expansive than the VME annotations 222 and may annotate the sensor data 228 outside of the indicated times when a user performed a VME task. For instance, in the exemplary illustration above, a user may sit with their hands in their lap in a manner similar to a VME task without intentionally performing a VME task. The second machine-learning algorithm may first identify periods of activities similar to VME or clinical tasks. The second machine-learning algorithm, or an additional machine-learning algorithm, may then generate annotations corresponding to contexts, performance, and other information related to the tasks to append to the sensor data 228. In this manner, the second machine-learning algorithm may produce a more accurate model of the behavior and actions of the user as well as providing additional levels of detail relating to disease progression, treatment effectiveness, or user status throughout a day, without the need to stop and perform a VME.

The user device 206 may generate a sensor data package 236. This may include the sensor data 228, annotations 232, and any VME data or contextual data from sensors of the user device 206 or of other sensors or devices.. In some examples, the sensor data package 236 may include other information relating to the VME. For example, images, videos, text, and the like may be bundled with the sensor data package 236. In some examples, the sensor data 228 and annotation 232 and any additional information that defines the context or status of the user may be identified by the user device 206, as described herein, and shared with the service provider 204 via a network such as the network 104. The sensor data package 236 may be useable by the user device 206 and/or the service provider 204 to assess how the user performed on the exam and throughout the free-living time period. In some examples, the service provider 204 may share aspects of the sensor exam data package 236 with other users such as medical professionals who are monitoring a clinical treatment, trial, disease progression, or other such tasks.

FIGS. 3, 6, and 7 illustrate example flow diagrams showing processes 300, 600, and 700 according to at least a few examples. These processes and any other processes described herein (e.g., the process 200) are illustrated as logical flow diagrams, each operation of which represents a sequence of operations that can be implemented in hardware, computer instructions, or a combination thereof. In the context of computer instructions, the operations may represent computer-executable instructions stored on one or more non-transitory computer-readable storage media that, when executed by one or more processors, perform the recited operations. Generally, computer-executable instructions include routines, programs, objects, components, data structures, and the like that perform particular functions or implement particular data types. The order in which the operations are described is not intended to be construed as a limitation, and any number of the described operations can be combined in any order and/or in parallel to implement the processes.

Additionally, some, any, or all of the processes described herein may be performed under the control of one or more computer systems configured with specific executable instructions and may be implemented as code (e.g., executable instructions, one or more computer programs, one or more applications) executing collectively on one or more processors, by hardware, or combinations thereof. As noted above, the code may be stored on a non-transitory computer-readable storage medium, for example, in the form of a computer program including a plurality of instructions executable by one or more processors.

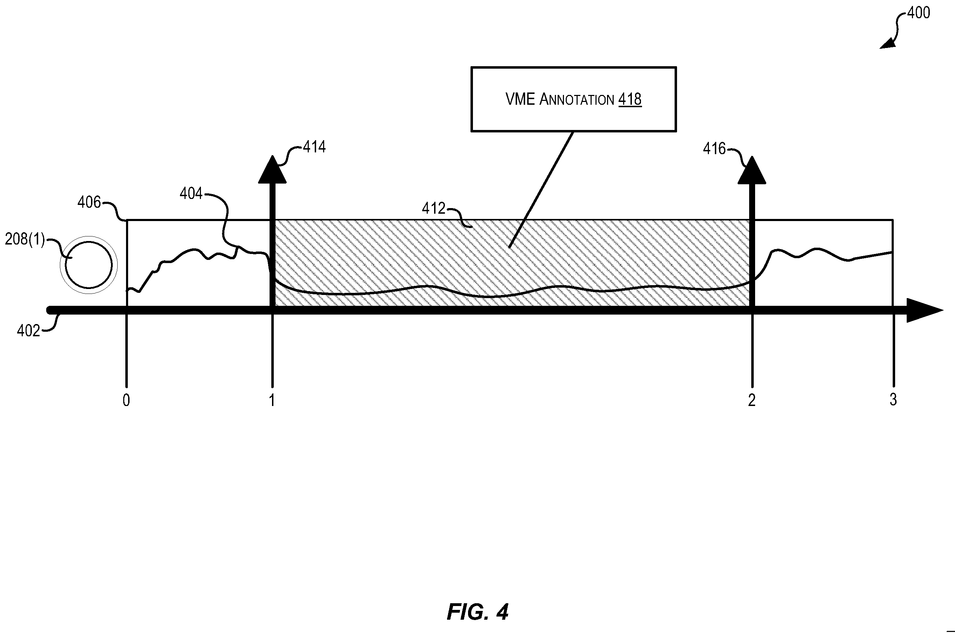

FIG. 3 illustrates an example flowchart illustrating the process 300 relating to implementing techniques relating to identifying activities and automatically generating annotations, according to at least one example. FIGS. 4-5 illustrate diagrams including various example sensors, example sensor data, and various devices. FIGS. 4-5 will be introduced with respect to FIG. 3. The process 300 is performed by the user device 102 of FIG. 1 or user device 206 of FIG. 2. The process 300 in particular corresponds to various approaches for identifying activities and generating annotations relating to the activities identified, according to various examples.

The process 300 begins at block 302 by the user device 102 determining a beginning of a time period of a possible activity of a particular type, such as a type of activity that may be performed as part of a VME. This may include determining the beginning based on sensor data information, including pattern recognition, or based on a user input indicating a start of a task. This may include using timestamps corresponding to user inputs at the user device 206. In some examples, the beginning of a time period of an activity similar to a VME task may be performed by segmenting portions of the sensor data and comparing the segmented data against historical examples of known sensor data from previous tasks. A machine-learning algorithm may provide a similarity score to one of a plurality of possible VME tasks that may be identified. In some examples, additional contextual clues may be used to narrow a potential list of tasks. For instance, some tasks may involve user movement and indicators such as position data, motion data, step tracking, and other such data from a user device, such as a smartphone, may be useful for identifying a subset of tasks related to a gait of a user. In another example, some tasks, such as related to identifying tremors, may require a stationary user and such location data may indicate a user is stationary. In addition, other sensor data related to a user's body position, pose, movement, acceleration, or any other such data may be used to narrow a list of potential tasks that may be identified.

At block 304, the process 300 includes the user device 102 accessing a historical sensor data profile associated with the particular type of activity and a sensor used to collect sensor data during the activity exam. This may include the user device 102 using a set of evaluation rules to determine which historical sensor data profile is appropriate as described above. In some examples, the historical sensor data profile may be accessed from memory of the user device 102 and/or requested from an external computing system. The evaluation rules may define, for a particular exam type, which profile is appropriate. The historical sensor data profile may be specific to a type of exam (e.g., sit and stand, hand movement, balance on one foot) and be specific to a type of sensor (e.g., accelerometer, gyroscope, heart rate monitor, etc.).

At block 306, the process 300 includes the user device 102 determining a difference between a portion of the signal data and a portion of the historical sensor data profile. The difference may be determined based on an output of a machine-learning algorithm, such as the first or second machine-learning algorithm described at blocks 240 and 242 of FIG. 2. As it can be assumed that the signal data will not be a perfect match with the historical sensor data profile, the user device 102 can compare the two to identify location(s) where the differences are minor and/or major, depending on a set of evaluation rules, or an overall similarity between the different profiles, e.g., based on an average difference between corresponding data points being below a threshold. For example, the evaluation rules may indicate that for a particular exam type and for this particular sensor, the user device 102 should expect to see large signal fluctuations during a "preparation time" and low signal fluctuations during the actual test. The historical signal profile may represent an averaged or learned depiction of these fluctuations.

At block 308, the process 300 includes the user device 102 using the historical signal profile to determine whether the differences are within some threshold. Small differences may indicate that the portion of the signal data is aligning with the historical signal profile. If the differences are too great, then the process 300 may return to the block 306 to continue to determine differences. If the differences are within the threshold, the process 300 may continue to block 310.

At block 310, the process 300 includes the user device 102 generating an annotation for the portion of the raw signal data. The annotation may include any of the annotations described herein and may be generated by a machine-learning algorithm as described above.

At block 312, the process 300 includes the user device 102 determining whether there are other sensors that can be used to generate additional annotations, for example describing one or more contexts of the environment or conditions at the time of the activity. If so, the process 300 returns to the block 304 and accesses a different historical sensor data profile associated with the particular type of virtual exam and a different sensor that collects different sensor data during the clinical exam. In some examples, the process 300 may be performed in parallel for multiple different sensors, rather than sequentially for each sensor. The annotations may additionally, in some examples, be generated as a result of data from multiple different sensors. For example, multiple sensors may describe a motion, position, and heart rate of the user that may all together be used to generate an annotation, or any other combination of data from various sensors may be used in conjunction to generate an annotation. Once any additional annotations have been generated, the process 300 proceeds to block 314, at which the process 300 includes providing the annotation(s) and the raw signal data to a storage device, of the user device 102 or of a remote system.

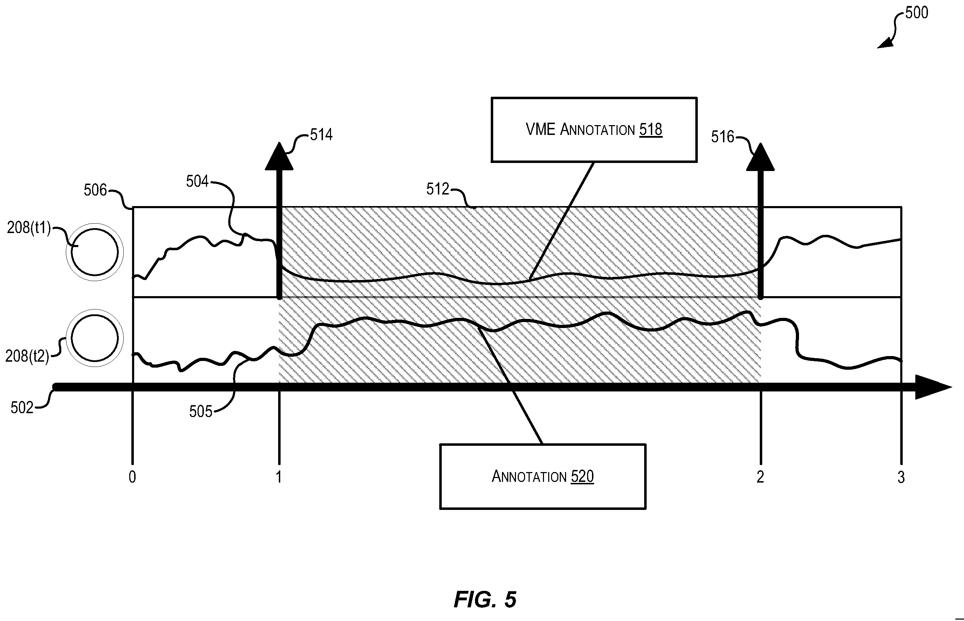

FIG. 4 illustrates a diagram 400 including an example sensor 208(1) and annotated sensor data, according to at least one example. The diagram 400 and the diagram 500 of FIG. 5 illustrate examples of sensor data that may be tagged with an annotations. The annotations may be identified by the machine learning algorithm based in similarities with previous tagged sets of data or otherwise identified with annotations. The annotations may be generated by a clinician or by an algorithm, as described above. The data may include accelerometer data and the tags may be associated with the data in a manner that identifies start and stop times of the relevant data and the corresponding annotation. In some examples, the annotation may be associated with a single set of data, such as depicted in FIG. 4 or may be associated with data from multiple sets of data, as in FIG. 5. The diagram 400 is an example of using a single sensor to track an activity performed during a clinical, virtual, or free-living task. The diagram 400 includes a timeline 402 and a representation of sensor data 404 obtained by the sensor 208(1) (e.g., one of the sensors 116-120 of FIG. 1) during some period 406, extending between t=0 and t=3. A profile of the sensor data 404 may vary at different times between t=0 and t=3. In some examples, the sampling rate of the sensor may vary between t=0 and t=3. The diagram 400 also includes timestamps 414 and 416 corresponding, respectively, to a user input or a machine-defined beginning and end of a virtual motor exam or activity. For example, after being prompted by the user device 102 to perform the virtual exam, the user may, at t=1, provide a command to the user device 102 in the form of a user interface selection, physical button, voice command, etc. to begin the virtual motor exam. The timestamp 414 may be generated by the user device 102 and associated with the sensor data 404 at that time. This may correspond to the block 302 of FIG. 3.

Between t=1 and t=2, the user may perform an activity, such as, for example, by sitting still and holding their hands in their lap. Thus, during this time, the sensor 208(1) (e.g., an accelerometer) shows very little movement. But as the virtual motor exam ends, the sensor data 404 shows more variation (e.g., between t=2 and t=3). In some examples, the window end 416 is not a determined value, but rather it is matched to the end of the task, which may be user-defined by selecting inputting at the user device that the exam has concluded or the end may be auto-defined (e.g., the virtual exam may run for a fixed period and may automatically end after the time has elapsed) or the end may be defined by a time when sensor data 404 or other sensor data indicates other activity by the user. The portion of the sensor data 404 within the context window 412 may be segmented from the other sensor data 404 and stored together with other information about the virtual motor exam, such as the VME annotation 418 (e.g., exam type, sensor type, window beginning, window end, task performed, predicted rating, predicted difficulty, pain level, and other such information), as described in block 310.

FIG. 5 illustrates a diagram 500 including example sensor data from the user device 102 at different points in time for identifying activities and generating annotations descriptive of activity performance, according to at least one example. The diagram 500 is an example of using a sensor 208 to gather data at two different periods of time and correlating the two different time segments of the sensor data to identify similar activities and generate annotations relating to the activity. The diagram 500 includes a timeline 502 and a representation of sensor data 504 obtained by the sensor 208(t1) at a first time and sensor data 505 obtained by the sensor 208(t2) at a second time, laid out such that a start and a stop are aligned during some period 506, extending between t=0 and t=3. Profiles of the sensor data 504 and 505 may vary at different times between t=0 and t=3. The diagram 500 also includes timestamps 514 and 516 corresponding, respectively, to a user input or a machine-defined beginning and end of a virtual motor exam at the first time, which may be known and clearly defined, while the second time may be during a free-living time period when data is not marked or labeled as part of a VME. This may correspond to the blocks 302 and 304 of FIG. 3 to select and identify the t1 window and the t2 window of sensor data. In FIG. 5, a context window 512 bounded by a window beginning at timestamp 514 and a window end 516 may be determined similar as described with respect to context window 412 of FIG. 4.

After selecting the time periods, as described with respect to blocks 302 and 304 of FIG. 3, a machine-learning algorithm may determine a level of similarity between the sensor data within the context window 512. When the similarity exceeds a predetermined threshold, the algorithm may generate an annotation 520 similar or identical to the VME annotation 518. In some examples, the sensor data 505 may be similar to more than one set of sensor data 504 associated with multiple VME annotations 518, in such examples, the annotation 520 may be generated as a compilation, average, or some combination of the VME annotations 518. In some examples, the average may be from a plurality of clinical providers, whose evaluations are all averaged to produce an annotation as a guide for the VME annotations.

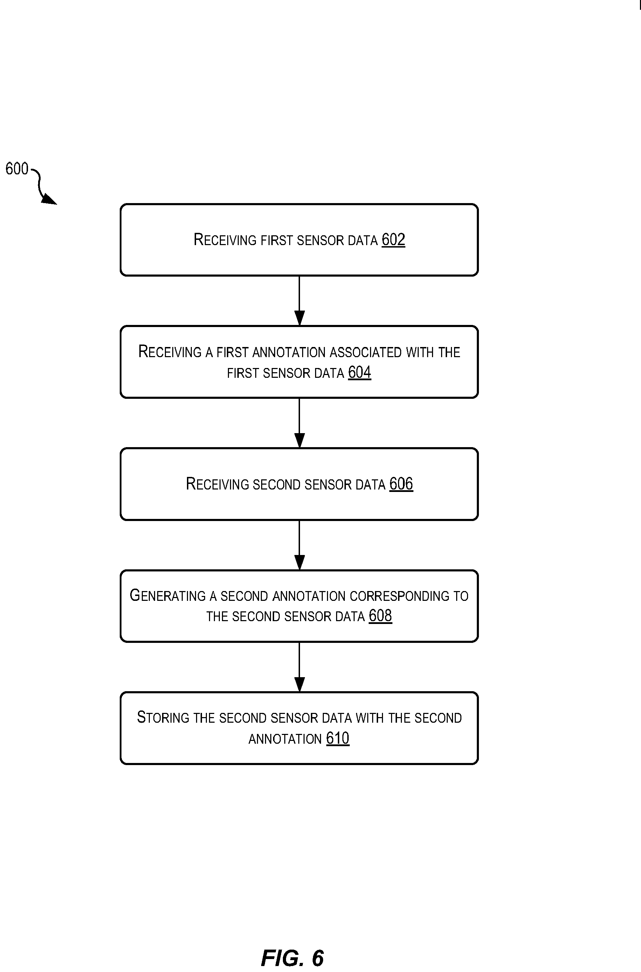

FIG. 6 illustrates an example flowchart illustrating the process 600 relating to implementing techniques relating to automatically generating annotations for sensor data from a wearable sensor, according to at least one example. The process 600 is performed by the user device 102 (FIG. 1) but may also be performed at a remote computer, such as the service provider 204 (FIG. 2). The process 600 in particular corresponds to various approaches for automatically generating annotations for sensor data from wearable sensors of user device 102 using machine-learning algorithms, according to various examples. In some examples, the user device 102 is a wearable user device such as a watch, ring, or other device described herein. Though the process 600 is described as performed on or by the user device 102, some or all of the actions of process 600 may be performed at a different or remote computing device including a linked computing device such as a smartphone or a remote computing device.

The process 600 begins at block 602 by the user device 102 receiving first sensor data. The first sensor data may be received from a sensor within the user device 102 and may be captured at a first time. The first time may correspond to a time when the user and the user device 102 are in a clinical setting, such as in a doctor's office or during a virtual motor exam with a remote clinician on a video call. The first sensor data may include information relating to the performance of one or more tasks by the user, such as accelerometer, position, or other such data, including motion, biometric, and other data gathered by sensors of user device 102. The virtual motor exam may include a series of tasks to evaluate motor function of a wearer of the user device 102. The sensor may include any one of the sensors described herein such as, for example, a gyroscope, an accelerometer, a photoplethysmography (PPG) sensor, a heart rate sensor, etc.

In some examples, the process 600 may further include the user device 102 receiving a first user input indicating a beginning of the virtual motor exam, generating a first timing indicator or annotation responsive to receiving the first user input and based on the first user input, receiving a second user input indicating an end of the virtual motor exam, and generating a second timing annotation responsive to receiving the second user input. In some examples the start and end times may be annotated by a clinician, as described at block 604 below.

At block 604, the process 600 includes the user device 102 receiving a first annotation associated with the first sensor data. The first annotation may include one or more types of data describing a type of task performed, a performance of the task, a subjective rating, clinician notes, a start and end time, and any other relevant information including subjective and objective notes corresponding to the performance of the task observed by the clinician.

At block 606, the process 600 includes the user device 102 receiving second sensor data at a second time, the second time different from the first time when the first sensor data is gathered at 602. The second sensor data may correspond to sensor data gathered outside of a clinical setting, including during a VME or during a typical day while a user wears the user device 102.

At block 608, the process 600 includes the user device 102 generating a second annotation corresponding to the second sensor data. Because the second sensor data may be captured outside of a clinical setting, the process 600 may attempt to identify data that represents activities that correspond to tasks that may be performed during a motor exam. Such activities may provide an opportunity to assess the wearer's performance of such a task, despite the wearer not consciously performing the task as part of a motor exam.

In this example, the second annotation is generated after identifying a task or action performed by a user as identified in on the second sensor data and based on identified tasks or actions and first annotations from the first sensor data. In some examples, generating the second annotation includes one or more additional steps corresponding to identifying tasks performed by a user and subsequently evaluating the performance of the tasks after the task is isolated in the second sensor data. The process 600 may, for example, include identifying a portion or segment of the second sensor data corresponding to a particular action or set of actions by a user, e.g., actions similar or identical to actions performed during the VME. The portion or segment of the second sensor data may be identified using the second machine-learning algorithm or the first machine-learning algorithm trained using sensor data gathered during a clinical visit or VME and tagged by a clinician or otherwise identified as corresponding to a motor exam task or other such activity. In this manner, the process 600 enables identification of tasks that a user is consciously or unconsciously performing without requiring explicit instructions as part of a VME. As an illustrative example, while a user is seated and watching television they may be holding their hands in their lap and holding their hands still, which may be similar to one task assigned as part of a motor exam to evaluate tremors in a user's hands. In another illustrative example, a user may be performing an everyday task, such as washing dishes or gardening, and while washing the dishes or gardening may incidentally perform motions that are identifiable based on the second sensor data as similar to a task from the motor exam, and identifiable by a machine-learning algorithm trained on VME data.

After identifying the portion of the second sensor data the second annotation may be generated and associated with the portion of the second sensor data. As part of the process of generating the second annotation, an activity or motion performed by the user is identified and the activity or motion is subsequently scored. The second annotation may store information similar to information stored in the first annotation, including descriptions of performance of tasks and subjective and objective feedback and measures. The second annotation is generated with a machine-learning algorithm trained using the first sensor data and the first annotation, including the machine-learning algorithms described herein. As described herein, the second annotation may be generated based on a similarity score between a historical example or by interpolation based on multiple previous historical examples. For instance, a task requiring the user to hold hands still in their lap may have varying results over time and receive different ratings, the machine-learning algorithm may identify that sensor data indicating higher amplitude or frequency of tremors may receive a lower rating while more steady sensor data (with respect to accelerometer data) may receive a higher rating.

After identifying a movement or activity performed by the user that is similar or identical to a task of the VME, the activity may be evaluated with the evaluation stored in the second annotation. The evaluation of the activity may be performed by the same machine-learning algorithm or may be performed by a separate evaluation algorithm. The evaluation may be triggered by the recognition or identification of one or more activities in the second sensor data. The evaluation of the portion of the second sensor data may be qualitative and quantitative including numerical scores or descriptions of performance on the task, with such descriptions generated by the machine-learning algorithm.

At block 610, the process 600 includes the user device 102 sending the second sensor data and the second annotation for storage at a memory of the user device 102 or at a remote server such as the service provider 204.

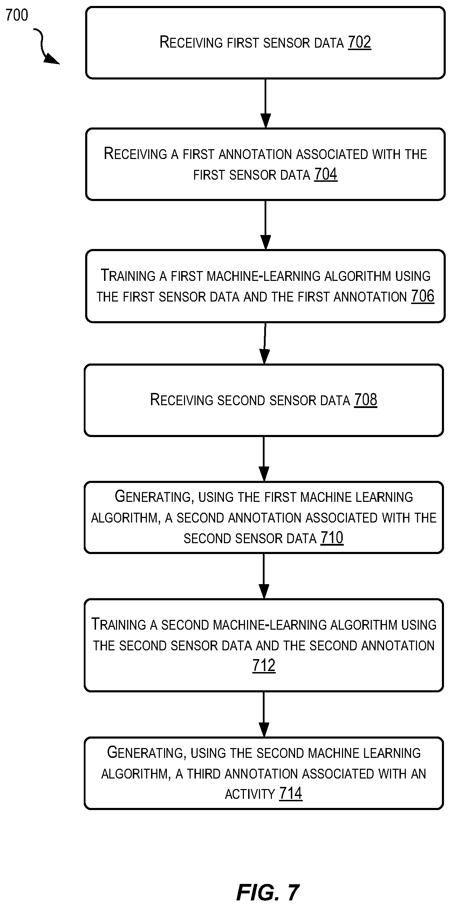

FIG. 7 illustrates an example flowchart illustrating a process 700 related to implementing techniques relating to training a machine-learning model to generate annotations for sensor data from a wearable sensor, according to at least one example. The process 700 includes development of a machine-learning algorithm to identify patterns and trends in symptoms and conditions in a free living situation. The process 700 may identify and learn patterns throughout days, weeks, months, and other periods of time to provide greater insights into the conditions and potential triggers for symptoms of a user. The process 700 is performed by the user device 102 (FIG. 1) but may also be performed at a remote computer, such as the service provider 204 (FIG. 2). The process 700 in particular corresponds to various approaches for training machine learning algorithms to identify activities and generate annotations based on previous sensor and annotation data. In some examples, the user device 102 is a wearable user device such as a watch, ring, or other device described herein. Though the process 700 is described as performed on or by the user device 102, some or all of the actions of process 700 may be performed at a different or remote computing device including a linked computing device such as a smartphone or a remote computing device. The process 700 may include the steps of process 600, for instance, process 700 may include steps 602-610 as part of steps 702-706.

The process 700 begins at block 702 by the user device 102 receiving first sensor data similar to block 602 of process 600. The first sensor data may be received from a sensor within the user device 102 and may be captured at a first time. The first time may correspond to a time when the user and the user device 102 are in a clinical setting, such as in a doctor's office or during a virtual motor exam with a remote clinician on a video call. The first sensor data may include information relating to the performance of one or more tasks by the user, such as accelerometer, position, or other such data, including motion, biometric, and other data gathered by sensors of user device 102. The virtual motor exam may include a series of tasks to evaluate motor function of a wearer of the user device 102. The sensor may include any one of the sensors described herein such as, for example, a gyroscope, an accelerometer, a photoplethysmography (PPG) sensor, a heart rate sensor, etc.

In some examples, the process 700 may further include the user device 102 receiving a first user input indicating a beginning of the virtual motor exam, generating a first timing indicator or annotation responsive to receiving the first user input and based on the first user input, receiving a second user input indicating an end of the virtual motor exam, and generating a second timing annotation responsive to receiving the second user input. In some examples the start and end times may be annotated by a clinician, as described at block 704 below.

At block 704, the process 700 includes the user device 102 receiving a first annotation associated with the first sensor data. The first annotation may include one or more types of data describing a type of task performed, a performance of the task, a subjective rating, clinician notes, a start and end time, or any other relevant information including subjective or objective notes corresponding to the performance of the task observed by the clinician.

At block 706, the process 700 includes training a first machine-learning algorithm using the first sensor data and the first annotation. The first machine-learning algorithm is trained based on annotation placed by clinicians or during and in response to the clinical exam as described with respect to FIG. 2 above, the clinical annotations being associated with particular portions of the first sensor data. The first machine-learning model may therefore be a rough model capable of producing annotations similar to those produced in the clinical annotations 216.

At block 708, the process 700 includes the user device 102 receiving second sensor data at a second time, the second time different from the first time when the first sensor data is gathered at 702. The second sensor data may correspond to sensor data gathered outside of a clinical setting, including during a VME or during a typical day while a user wears the user device 102.

At block 710, the process 700 includes the user device 102 generating a second annotation corresponding to the second sensor data using the first machine-learning algorithm. The second annotation may store information similar to information stored in the first annotation, including descriptions of performance of tasks and subjective and objective feedback and measures. The second annotation is generated with the first machine-learning algorithm trained using the first sensor data and the first annotation, including the machine-learning algorithms described herein.

At block 712, the process 700 includes training a second machine-learning algorithm using the second sensor data and the second annotation. The second machine-learning algorithm may be of a similar or identical type to the first machine-learning algorithm described above, and with the additional training data from the second sensor data and the second annotations, the second machine-learning algorithm may produce additional annotations, more accurate annotations, and further be capable of identifying activities associated with the VME, or other tasks, without input from the user indicating the start of a task. The second machine-learning algorithm may receive inputs of the sensor data, time, activity data, or any other suitable data corresponding to actions, activities, and free living environments. The second machine-learning algorithm may be trained using the second annotations, the sensor data, and any additional data, such as the time of day, location data, and activity data, such as to recognize correlations between the sensor data and other aspects of their daily lives. For example, the second machine learning algorithm may receive sensor data and then annotations from the first machine-learning algorithm, along with time information. The second machine-learning algorithm may encode such data into a latent space, which may enable it to populate the latent space over time and develop the ability to predict user activity based on the encoded data. For example, over time, the second machine-learning algorithm may develop a latent space that indicates that the user has more significant tremors in the morning, but that they abate over the course of the day, or that the tremors are associated with particular movements. In this manner, the second machine-learning algorithm may be trained to identify patterns and long-term trends in symptoms and conditions for the user.

At block 714, the process 700 includes generating, using the second machine-learning algorithm, a third annotation associated with an activity. The second machine-learning algorithm may enable identification of longer term patterns, trends, and correlations between certain activities, times of days, and other triggers for symptoms or conditions. The second machine-learning algorithm may generate third annotations corresponding to sensor data gathered while the user wears the user device 102 but outside of a clinical or VME setting and may therefore provide insights into longer term trends, triggers, or other patterns associated with various symptoms of a user. This is enabled by identifying longer terms trends and triggers by identifying portions of sensor data identified by similarity with previously tagged sensor data and subsequently identifying further data over a longer period of time to identify additional triggers for particular symptoms or times of day when a condition may be especially difficult for a user. The third annotations may be more expansive than the first and second annotations and may annotate the sensor data outside of the indicated times when a user performed a VME task. For instance, in the exemplary illustration above, a user may sit with their hands in their lap in a manner similar to a VME task without intentionally performing a VME task. The second machine-learning algorithm may first identify periods of activities similar to VME or clinical tasks. The second machine-learning algorithm, or an additional machine-learning algorithm, may then generate the third annotations corresponding to contexts, performance, and other information related to the tasks to append to the sensor data. In this manner, the second machine-learning algorithm may produce a more accurate model of the behavior and actions of the user as well as providing additional levels of detail relating to disease progression, treatment effectiveness, or user status throughout a day, without the need to stop and perform a VME.

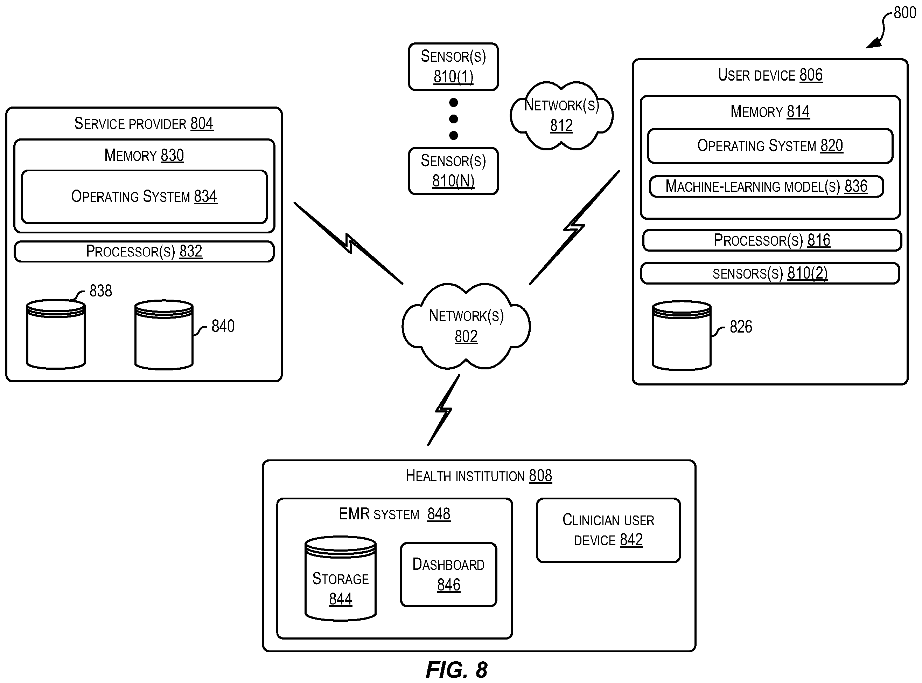

FIG. 8 illustrates an example architecture 800 or environment configured to implement techniques relating to identifying activities and annotating sensor data associated with the activities, according to at least one example. For example, the architecture 800 enables data sharing between the various entities of the architecture, at least some of which may be connected via one or more networks 802, 812. For example, the example architecture 800 may be configured to enable a user device 806 (e.g., the user device 102 or 206), a service provider 804 (e.g., the service provider 204, sometimes referred to herein as a remote server, service-provider computer, and the like), a health institution 808, and any other sensors 810 (e.g., the sensors 116-120 and 130) to share information. In some examples, the service provider 804, the user device 806, the health institution 808, and the sensors 810(1)-810(N) may be connected via one or more networks 802 and/or 812 (e.g., via Bluetooth, WiFi, the Internet, cellular, or the like). In the architecture 800, one or more users may utilize a different user device to manage, control, or otherwise utilize the user device 806 via the one or more networks 812 (or other networks). Additionally, in some examples, the user device 806, the service provider 804, and the sensors 810 may be configured or otherwise built as a single device such that the functions described with respect to the service provider 804 may be performed by the user device 806 and vice versa.

In some examples, the networks 802, 812 may include any one or a combination of many different types of networks, such as cable networks, the Internet, wireless networks, cellular networks, satellite networks, other private and/or public networks, or any combination thereof. While the illustrated example represents the user device 806 accessing the service provider 804 via the networks 802, the described techniques may equally apply in instances where the user device 806 interacts with the service provider 804 over a landline phone, via a kiosk, or in any other manner. It is also noted that the described techniques may apply in other client/server arrangements (e.g., set-top boxes), as well as in non-client/server arrangements (e.g., locally stored applications, peer-to-peer configurations).

As noted above, the user device 806 may be configured to collect and/or manage user activity data potentially received from the sensors 810. In some examples, the user device 806 may be configured to provide health, fitness, activity, and/or medical data of the user to a third- or first-party application (e.g., the service provider 804). In turn, this data may be used by the service provider 804 in implementing techniques described herein.

The user device 806 may be any type of computing device, such as, but not limited to, a mobile phone, a smartphone, a personal digital assistant (PDA), a wearable device (e.g., ring, watch, necklace, sticker, belt, shoe, shoe attachment, belt-clipped device) an implantable device, or the like. In some examples, the user device 806 may be in communication with the service provider 804; the sensors 810; and/or the health institution via the networks 802, 812; or via other network connections.

The sensors 810 may be standalone sensors or may be incorporated into one or more devices. In some examples, the sensors 810 may collect sensor data that is shared with the user device 806 and related to implementing the techniques described herein. For example, the user device 806 may be a primary user device (e.g., a smartphone) and the sensors 810 may be sensor devices that are external from the user device 806 and can share sensor data with the user device 806. For example, external sensors 810 may share information with the user device 806 via the network 812 (e.g., via Bluetooth or other near-field communication protocol). In some examples, the external sensors 810 include network radios that allow them to communicate with the user device 806 and/or the service provider 804. The user device 806 may include one or more applications for managing the remote sensors 810. This may enable pairing with the sensors 810, data reporting frequencies, data processing of the data from the sensors 810, data alignment, and the like.

The sensors 810 may be attached to various parts of a human body (e.g., feet, legs, torso, arms, hands, neck, head, eyes) to collect various types of information, such as activity data, movement data, or heart rate data. The sensors 810 may include accelerometers, respiration sensors, gyroscopes, PPG sensors, pulse oximeters, electrocardiogram (ECG) sensors, electromyography (EMG) sensors, electroencephalography (EEG) sensors, global positioning system (GPS) sensors, auditory sensors (e.g., microphones), ambient light sensors, barometric altimeters, electrical and optical heart rate sensors, and any other suitable sensor designed to obtain physiological data, physical condition data, and/or movement data of a user.

In one illustrative configuration, the user device 806 may include at least one memory 814 and one or more processing units (or processor(s)) 816. The processor(s) 816 may be implemented as appropriate in hardware, computer-executable instructions, firmware, or combinations thereof. Computer-executable instruction or firmware implementations of the processor(s) 816 may include computer-executable or machine-executable instructions written in any suitable programming language to perform the various functions described. The user device 806 may also include geo-location devices (e.g., a GPS device or the like) for providing and/or recording geographic location information associated with the user device 806. The user device 806 also includes one or more sensors 810(2), which may be of the same type as those described with respect to the sensors 810.

Depending on the configuration and type of the user device 806, the memory 814 may be volatile (such as random-access memory (RAM)) and/or non-volatile (such as read-only memory (ROM), flash memory). While the volatile memory described herein may be referred to as RAM, any volatile memory that would not maintain data stored therein once unplugged from a host and/or power would be appropriate.

Both the removable and non-removable memory 814 are examples of non-transitory computer-readable storage media. For example, non-transitory computer-readable storage media may include volatile or non-volatile, removable or non-removable media implemented in any method or technology for storage of information such as computer-readable instructions, data structures, program modules, or other data. The memory 814 is an example a of non-transitory computer-readable storage media or non-transitory computer-readable storage device. Additional types of computer storage media that may be present in the user device 806 may include, but are not limited to, PRAM, SRAM, DRAM, RAM, ROM, EEPROM, flash memory or other memory technology, CD-ROM, DVD or other optical storage, magnetic cassettes, magnetic tape, magnetic disk storage or other magnetic storage devices, or any other medium that can be used to store the desired information and that can be accessed by the user device 806. Combinations of any of the above should also be included within the scope of non-transitory computer-readable storage media. Alternatively, computer-readable communication media may include computer-readable instructions, program modules, or other data transmitted within a data signal, such as a carrier wave, or other transmission. However, as used herein, computer-readable storage media does not include computer-readable communication media.

Turning to the contents of the memory 814 in more detail, the memory 814 may include an operating system 820 and/or one or more application programs or services for implementing the features disclosed herein. The user device 806 also includes one or more machine-learning models 836 representing any suitable predictive model. The machine-learning models 836 may be utilized by the user device 806 to identify activities and generate annotations, as described herein.

The service provider 804 may also include a memory 824 including one or more applications programs or services for implementing the features disclosed herein. In this manner, the techniques described herein may be implemented by any one, or a combination of more than one, of the computing devices (e.g., the user device 806 and the service provider 804).

The user device 806 also includes a datastore that includes one or more databases or the like for storing data such as sensor data and static data. In some examples, the databases 826 and 828 may be accessed via a network service.

The service provider 804 may also be any type of computing device, such as, but not limited to, a mobile phone, a smartphone, a PDA, a laptop computer, a desktop computer, a thin-client device, a tablet computer, a wearable device, a server computer, or a virtual machine instance. In some examples, the service provider 804 may be in communication with the user device 806 and the health institution 808 via the network 802 or via other network connections.

In one illustrative configuration, the service provider 804 may include at least one memory 830 and one or more processing units (or processor(s)) 832. The processor(s) 832 may be implemented as appropriate in hardware, computer-executable instructions, firmware, or combinations thereof. Computer-executable instruction or firmware implementations of the processor(s) 832 may include computer-executable or machine-executable instructions written in any suitable programming language to perform the various functions described.

The memory 830 may store program instructions that are loadable and executable on the processor(s) 832, as well as data generated during the execution of these programs. Depending on the configuration and type of service provider 804, the memory 830 may be volatile (such as RAM) and/or non-volatile (such as ROM, flash memory). While the volatile memory described herein may be referred to as RAM, any volatile memory that would not maintain data stored therein once unplugged from a host and/or power would be appropriate. Both the removable and non-removable memory 830 are additional examples of non-transitory computer-readable storage media.

Turning to the contents of the memory 830 in more detail, the memory 830 may include an operating system 834 and/or one or more application programs or services for implementing the features disclosed herein.

The service provider 804 also includes a datastore that includes one or more databases or the like for storing data, such as sensor data and static data. In some examples, the databases 838 and 840 may be accessed via a network service.

Turning now to the health institution 808, while depicted as a single entity, the health institution 808 may represent multiple health institutions. The health institution 808 includes an EMR system 848, which is accessed via a dashboard 846 (e.g., by a user using a clinician user device 842). In some examples, the EMR system 848 may include a record storage 844 and a dashboard 846. The record storage 844 may be used to store health records of users associated with the health institution 808. The dashboard 846 may be used to read and write the records in the record storage 844. In some examples, the dashboard 846 is used by a clinician to manage disease progression for a user population including a user who operates the user device 102. The clinician may operate the clinician user device 842 to interact with the dashboard 846 to view results of virtual motor exams on a user-by-user basis, on a population of user basis, etc. In some examples, the clinician may use the dashboard 846 to "push" an exam to the user device 102.

Unless specifically stated otherwise, it is appreciated that throughout this specification discussions utilizing terms such as "processing," "computing," "calculating," "determining," and "identifying" or the like refer to actions or processes of a computing device, such as one or more computers or a similar electronic computing device or devices, that manipulate or transform data represented as physical electronic or magnetic quantities within memories, registers, or other information storage devices, transmission devices, or display devices of the computing platform.

The system or systems discussed herein are not limited to any particular hardware architecture or configuration. A computing device can include any suitable arrangement of components that provides a result conditioned on one or more inputs. Suitable computing devices include multipurpose microprocessor-based computing systems accessing stored software that programs or configures the computing system from a general-purpose computing apparatus to a specialized computing apparatus implementing one or more embodiments of the present subject matter. Any suitable programming, scripting, or other type of language or combinations of languages may be used to implement the teachings contained herein in software to be used in programming or configuring a computing device.

Embodiments of the methods disclosed herein may be performed in the operation of such computing devices. The order of the blocks presented in the examples above can be varied-for example, blocks can be reordered, combined, and/or broken into sub-blocks. Certain blocks or processes can be performed in parallel.

Conditional language used herein, such as among others, "can," "could," "might," "may," "e.g.," and the like, unless specifically stated otherwise or otherwise understood within the context as used, is generally intended to convey that certain examples include, while other examples do not include, certain features, elements, and/or steps. Thus, such conditional language is not generally intended to imply that features, elements, and/or steps are in any way required for one or more examples or that one or more examples necessarily include logic for deciding, with or without author input or prompting, whether these features, elements and/or steps are included or are to be performed in any particular example.

Disjunctive language such as the phrase "at least one of X, Y, or Z," unless specifically stated otherwise, is otherwise understood within the context as used in general to present that an item, term, etc., may be either X, Y, or Z, or any combination thereof (e.g., X, Y, and/or Z). Thus, such disjunctive language is not generally intended to, and should not, imply that certain examples require at least one of X, at least one of Y, or at least one of Z to each be present.

Use herein of the word "or" is intended to cover inclusive and exclusive OR conditions. In other words, "A or B or C" includes any or all of the following alternative combinations as appropriate for a particular usage: A alone; B alone; C alone; A and B only; A and C only; B and C only; and all three of A and B and C.

The use of the terms "a," "an," and "the" and similar referents in the context of describing the disclosed examples (especially in the context of the following claims) are to be construed to cover both the singular and the plural, unless otherwise indicated herein or clearly contradicted by context. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. The use of "adapted to" or "configured to" herein is meant as open and inclusive language that does not foreclose devices adapted to or configured to perform additional tasks or steps. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. Additionally, the use of "based on" is meant to be open and inclusive, in that a process, step, calculation, or other action "based on" one or more recited conditions or values may, in practice, be based on additional conditions or values beyond those recited. Similarly, the use of "based at least in part on" is meant to be open and inclusive, in that a process, step, calculation, or other action "based at least in part on" one or more recited conditions or values may in practice be based on additional conditions or values beyond those recited. Headings, lists, and numbering included herein are for ease of explanation only and are not meant to be limiting.

The various features and processes described above may be used independently of one another or may be combined in various ways. All possible combinations and subcombinations are intended to fall within the scope of the present disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed examples. Similarly, the example systems and components described herein may be configured differently than described. For example, elements may be added to, removed from, or rearranged compared to the disclosed examples.

## Claims

1. A computer-implemented method, comprising:
receiving sensor data from a wearable sensor system worn by a user during user activity in a free-living environment throughout an extended period of time;
determining, based on the sensor data, that the user activity corresponds with a clinical exam activity during a time period within the extended period of time; and
generating, using a machine learning algorithm and by the wearable sensor system, an annotation indicative of a predicted clinical exam score of the clinical exam activity, wherein prior to generating the annotation, the machine learning algorithm is trained using clinical exam data and clinical exam annotations indicating a performance of the clinical exam activity by a subject.

2. The computer-implemented method of claim 1, wherein the predicted clinical exam score comprises a motor exam to evaluate progression of a disease affecting user motor control.

3. The computer-implemented method of claim 1 or 2, wherein the predicted clinical exam score provides a quantitative score for the performance of the clinical exam activity based on the sensor data.

4. The computer-implemented method of any preceding claim, wherein the annotation comprises a predicted subjective user rating during performance of the user activity.

5. The computer-implemented method of any preceding claim, wherein the annotation is generated using the wearable sensor system at a time of receiving the sensor data.

6. The computer-implemented method of any preceding claim, wherein determining that the user activity corresponds with the clinical exam activity comprises receiving an input at a user device indicating that a user is beginning a virtual motor exam.

7. The computer-implemented method of any preceding claim, further comprising receiving a confirmation of the annotation via a user interface of the wearable sensor system.

8. A computer system comprising one or more processors configured to perform the method of any one of claims 1 to 7.

9. A computer-readable storage medium comprising instructions which, when executed by a computer, cause the computer to carry out the method of any one of claims 1 to 7.

## Patentansprüche

1. Computerimplementiertes Verfahren, umfassend:
Empfangen von Sensordaten von einem tragbaren Sensorsystem, das durch einen Benutzer während einer Benutzeraktivität in einer freien Lebensumgebung über einen längeren Zeitraum hinweg getragen wird;
Bestimmen, dass die Benutzeraktivität einer klinischen Untersuchungsaktivität während eines Zeitraums innerhalb des längeren Zeitraums entspricht, basierend auf den Sensordaten; und
unter Verwendung eines maschinellen Lernalgorithmus und durch das tragbare Sensorsystem Erzeugen einer Annotation, die einen vorhergesagten klinischen Score-Wert der klinischen Untersuchungsaktivität angibt, wobei vor dem Erzeugen der Annotation der maschinelle Lernalgorithmus unter Verwendung von klinischen Untersuchungsdaten und klinischen Untersuchungsannotationen, die eine Leistungsfähigkeit der klinischen Untersuchungsaktivität durch ein Individuum angeben, trainiert wird.

2. Computerimplementiertes Verfahren nach Anspruch 1, wobei der vorhergesagte klinische Score-Wert eine motorische Untersuchung zur Bewertung des Fortschreitens einer Erkrankung, welche die motorische Steuerung des Benutzers beeinträchtigt, umfasst.

3. Computerimplementiertes Verfahren nach Anspruch 1 oder 2, wobei der vorhergesagte klinische Score-Wert einen quantitativen Score-Wert für die Ausführung der klinischen Untersuchungsaktivität basierend auf den Sensordaten bereitstellt.

4. Computerimplementiertes Verfahren nach einem der vorangegangenen Ansprüche, wobei die Annotation eine vorhergesagte subjektive Benutzereinstufung während der Ausführung der Benutzeraktivität umfasst.

5. Computerimplementiertes Verfahren nach einem der vorangegangenen Ansprüche, wobei die Annotation unter Verwendung des tragbaren Sensorsystems zu einem Zeitpunkt des Empfangens der Sensordaten erzeugt wird.

6. Computerimplementiertes Verfahren nach einem der vorangegangenen Ansprüche, wobei das Bestimmen, dass die Benutzeraktivität der klinischen Untersuchungsaktivität entspricht, das Empfangen einer Eingabe an einer Benutzervorrichtung umfasst, welche angibt, dass ein Benutzer eine virtuelle motorische Untersuchung beginnt.

7. Computerimplementiertes Verfahren nach einem der vorangegangenen Ansprüche, ferner umfassend das Empfangen einer Bestätigung der Annotation über eine Benutzeroberfläche des tragbaren Sensorsystems.

8. Computersystem, umfassend einen oder mehrere Prozessoren, die konfiguriert sind, um ein Verfahren nach einem der Ansprüche 1 bis 7 durchzuführen.

9. Computerlesbares Speichermedium, umfassend Befehle, die, wenn sie durch einen Computer ausgeführt werden, bewirken, dass der Computer ein Verfahren nach einem der Ansprüche 1 bis 7 ausführt.

## Revendications

1. Procédé mis en œuvre par ordinateur, comprenant les étapes consistant à :
recevoir des données de capteur à partir d'un système de capteur portable porté par un utilisateur pendant une activité d'utilisateur dans un environnement de vie libre pendant une période de temps prolongée ;
déterminer, sur la base des données de capteur, que l'activité d'utilisateur correspond à une activité d'examen clinique pendant une période de temps dans la période de temps prolongée ; et
générer, en utilisant un algorithme d'apprentissage automatique et par l'intermédiaire du système de capteur portable, une annotation indicative d'un score d'examen clinique prédit de l'activité d'examen clinique, dans lequel avant de générer l'annotation, l'algorithme d'apprentissage automatique est entraîné en utilisant des données d'examen clinique et des annotations d'examen clinique indiquant une performance de l'activité d'examen clinique par un sujet.

2. Procédé mis en œuvre par ordinateur selon la revendication 1, dans lequel le score d'examen clinique prédit comprend un examen de motricité pour évaluer la progression d'une maladie affectant la commande de motricité de l'utilisateur.

3. Procédé mis en œuvre par ordinateur selon la revendication 1 ou 2, dans lequel le score d'examen clinique prédit fournit un score quantitatif pour la réalisation de l'activité d'examen clinique sur la base des données de capteur.

4. Procédé mis en œuvre par ordinateur selon l'une quelconque des revendications précédentes, dans lequel l'annotation comprend une évaluation subjective prédite de l'utilisateur pendant l'exécution de l'activité d'utilisateur.

5. Procédé mis en œuvre par ordinateur selon l'une quelconque des revendications précédentes, dans lequel l'annotation est générée en utilisant le système de capteur portable au moment de la réception des données de capteur.

6. Procédé mis en œuvre par ordinateur selon l'une quelconque des revendications précédentes, dans lequel la détermination que l'activité d'utilisateur correspond à l'activité d'examen clinique comprend la réception d'une entrée au niveau d'un dispositif utilisateur indiquant qu'un utilisateur commence un examen de motricité virtuel.

7. Procédé mis en œuvre par ordinateur selon l'une quelconque des revendications précédentes, comprenant en outre une réception d'une confirmation de l'annotation via une interface utilisateur du système de capteur portable.

8. Système informatique comprenant un ou plusieurs processeurs configurés pour exécuter le procédé selon l'une quelconque des revendications 1 à 7.

9. Support de stockage lisible par ordinateur comprenant des instructions qui, lorsqu'elles sont exécutées par un ordinateur, amènent l'ordinateur à exécuter le procédé selon l'une quelconque des revendications 1 à 7.
